(19) Europäisches Patentamt
European Patent Office
Office européen des brevets

(11) Veröffentlichungsnummer: **0 000 947**
**B2**

# (12) NEUE EUROPÄISCHE PATENTSCHRIFT

(45) Veröffentlichungstag der neuen Patentschrift: **10.10.84**

(21) Anmeldenummer: **78100750.5**

(22) Anmeldetag: **25.08.78**

(51) Int. Cl.³: **C 07 D 211/46,** C 07 D 211/60, C 07 H 15/12, C 07 D 498/04, A 61 K 31/70, A 23 K 1/16, A 61 K 31/445, C 07 D 403/12, C 07 D 403/06, C 07 D 405/06, A 23 L 1/30 // C07H15/18, C07H15/12, C07H15/14, (C07D498/04, 265/00, 221/00)

(54) **Neue Derivate von 3,4,5-Trihydroxypiperidin, Verfahren zu ihrer Herstellung und sie enthaltende Arznei- und Futtermittel.**

(30) Priorität: **27.08.77 DE 2738717**
**24.12.77 DE 2758025**

(43) Veröffentlichungstag der Anmeldung: **07.03.79 Patentblatt 79/5**

(45) Bekanntmachung des Hinweises auf die Patenterteilung: **14.01.81 Patentblatt 81/2**

(45) Bekanntmachung des Hinweises auf die Entscheidung über den Einspruch: **10.10.84 Patentblatt 84/41**

(84) Benannte Vertragsstaaten: **BE CH DE FR GB LU NL SE**

(56) Entgegenhaltungen:
**DE - A - 2 656 602**
**DE - A - 2 660 848**
**FR - A - 2 336 941**

**CHEMICAL ABSTRACTS, 66, 1967 65781n**
**CHEMISCHE BERICHTE 100 (8), 1967 Seiten 2467-2478**
**DICTIONARY OF ORGANIC COMPOUNDS Eyre & Spottiswoode Publichers Ltd., 1974 London Fourth Edition Tenth and Cumulative Supplement Site 723 "Nojirimycin"**

(73) Patentinhaber: **BAYER AG, Konzernverwaltung RP Patentabteilung, D-5090 Leverkusen 1 Bayerwerk (DE)**

(72) Erfinder: **Junge, Bodo, Dr., Wilkhausstrasse 123, D-5600 Wuppertal 2 (DE)**
Erfinder: **Krause, Hans Peter Dr., Wilkhausstrasse 107, D-5600 Wuppertal-2 (DE)**
Erfinder: **Müller, Lutz, Dr., Kronprinzenallee 111, D-5600 Wuppertal-1 (DE)**
Erfinder: **Puls, Walter, Dr., In den Birken 75, D-5600 Wuppertal 1 (DE)**



BUNDESDRUCKEREI BERLIN

## Neue Derivate von 3,4,5-Trihydroxypiperidin, Verfahren zu ihrer Herstellung und sie enthaltende Arznei- und Tierfuttermittel

Die vorliegende Erfindung betrifft neue Derivate von 3,4,5-Trihydroxypiperidin, mehrere Verfahren zu ihrer Herstellung, diese Verbindung enthaltende Arzneimittel, diese Verbindungen zur Anwendung bei der Behandlung von Diabetes, Hyperlipämie und Adipositas, wowie die Verwendung in der Tierernährung zur Beeinflussung des Fleisch/Fettverhältnisses zugunsten des Fleischanteils.

Die neuen Derivate lassen sich durch die Formel I wiedergeben

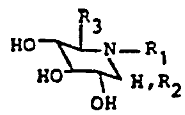

(I)

in der

$R_1$ H oder einen gegebenenfalls substituierten, geradkettigen, verzweigten oder cyclischen gesättigten oder ungesättigten aliphatischen Kohlenwasserstoffrest darstellt,

$R_2$ $-H$, $-OR'$, $-SR'$, $-N{<}^{R'}_{R''}$, $NR'R''-CH_2-$, $-COOR'$, $HO-CH_2-$, $R'CO-NR''CH_2-$,

$R'SO_2-NR''CH_2-$, $R'-NH-\underset{O}{\underset{\|}{C}}-NH-CH_2-$, $R'-NH-\underset{S}{\underset{\|}{C}}-NH-CH_2-$, $R'-O-\underset{O}{\underset{\|}{C}}-NH-CH_2-$,

$-SO_3H$, $-CN$ oder $-CONR'R''$ bedeutet und

$R_3$ die für $R_1$ gegebene Bedeutung annehmen kann wobei

$R'$, $R''$ Wasserstoff, einen gegebenenfalls substituierten geradkettigen, verzweigten oder cyclischen gesättigten oder ungesättigten aliphatischen Kohlenwasserstoffrest oder einen gegebenenfalls substituierten aromatischen oder heterocyclischen Rest bedeutet und wobei

$R_1$ nicht Wasserstoff ist, wenn

a) $R_2$ Wasserstoff oder OH und $R_3$ $CH_2OH$

b) $R_2$ Wasserstoff, OH, $SO_3H$, CN oder $CH_2NH_2$ und $R_3$ Wasserstoff und

c) $R_2$ OH und $R_3$ $CH_2NH_2$ bedeuten.

und wobei $R_1$ nicht $C_1$—$C_4$-Alkyl ist, wenn $R_2 = H$ und $R_3 = CH_2OH$ ist.

Die Erfindung betrifft darüber hinaus auch pharmazeutisch annehmbare Salze der Verbindungen der Formel I wie Chloride, Sulfate, Acetate, Carbonate, Oxalate usw., und Bio-Vorläufer, wobei unter Bio-Vorläufer Verbindungen verstanden werden, deren Struktur sich von der aktiven Verbindung unterscheidet, die jedoch nach Verabreichung an Mensch oder Tier im Körper des Patienten in die aktive Verbindung umgewandelt werden.

Bevorzugt bedeuten $R_1$, $R'$, $R''$ einen Alkylrest mit 1 bis 30 insbesondere 1 bis 18 C-Atomen, einen Alkenylrest oder Alkinyl- oder tricyclischen Rest mit 3 bis 10 C-Atomen, der gesättigt, einfach oder doppelt ungesättigt sein kann, einen Arylrest mit 6 oder 10 C-Atomen, einen heterocyclischen Rest mit 3 bis 8 insbesondere 3 bis 6 Ringgliedern, der 1, 2, 3 oder 4 Heteroatome, insbesondere N, O, S enthalten kann und an dem ein Benzolring oder ein weiterer Heterozyklus der genannten Art ankondensiert sein kann, wobei die genannten Reste 1 bis 5 insbesondere 1, 2 oder 3 Substituenten tragen können.

Als Substituenten für Alkyl seien beispielhaft aufgeführt: Hydroxy, Alkoxy mit vorzugsweise 1 bis 4 Kohlenstoffatomen, insbesondere Methoxy und Aethoxy; Acyloxy, wobei der Acylrest von aliphatischen Carbonsäuren mit 1 bis 7 C-Atomen, aromatischen Carbonsäuren insbesondere Phenylcarbonsäuren, die im Phenylrest durch —OH, -Halogen, insbesondere F, Cl, Br, $C_1$—$C_4$-Alkyl, $C_1$—$C_4$-Alkoxy, Nitro und/oder Amino substituiert sein können, heterocyclischen Carbonsäuren, die sich von 5- oder 6-gliedrigen Heterocyclen ableiten, die 1 bis 3 Heteroatome (N,O,S) enthalten und im heterocyclischen Ring durch $C_1$—$C_4$-Alkyl, Chlor, Brom, Amino substituiert sein können, abgeleitet ist; Amino, Monoalkylamino und Dialkylamino mit vorzugsweise 1 bis 4 Kohlenstoffatomen je Alkylrest, insbesondere Monomethylamino, Monoäthylamino, Dimethylamino und Diäthylamino, Monoacylamino, wobei der Acylrest von aliphatischen Carbonsäuren mit 1 bis 7 C-Atomen, aromatischen Carbonsäuren insbesondere Phenylcarbonsäuren, die im Phenylrest durch —OH, -Halogen, insbesondere F, Cl, Br, $C_1$—$C_4$-Alkyl, $C_1$—$C_4$-Alkoxy, Nitro und/oder Amino substituiert sein können, heterocyclischen Carbonsäuren, die sich von 5- oder 6-gliedrigen Heterocyclen ableiten, die 1 bis 3 Heteroatome (N, O, S) enthalten und im heterocyclischen Ring durch $C_1$—$C_4$-Alkyl, Chlor, Brom, Amino substituiert sein können, abgeleitet ist; Mercapto, Alkylthio mit vorzugsweise 1 bis 4 Kohlenstoffatomen, insbesondere Methylthio und Aethylthio; Halogen, vorzugsweise Fluor, Chlor und Brom; Alkylcarbonyl mit vorzugsweise 1 bis 4 Kohlenstoffatomen im Alkylrest; Carboxy, Nitro, Cyan, die Aldehydfunktion, die Sulfonsäuregruppe; sowie heterocyclische Reste der oben genannten Art, insbesondere auch von Zuckern, ganz besonders von Hexosen oder Pentosen abgeleitete heterocyclische Reste, die direkt über ein Ringatom oder über eine —O—, —S— oder —NH-Brücke mit dem Alkylrest verbunden sein können.

Beispiele für heterocyclische Substituenten der Alkylreste sind: Phthalimido, Pyridyl, Thienyl, Furyl, Isoxazolyl, Thiazolyl, Glucopyranosyl, Ribofuranosyl, Oxiranyl u. dgl. Des weiteren eignen sich als Substituenten der Alkylreste aromatische Reste wie Naphthyl und insbesondere Phenyl, die einen oder mehrere, vorzugsweise 1 bis 3 gleiche oder verschiedene Substituenten aus der Reihe —OH, —$NH_2$,

$C_1$—$C_4$-Alkyl-NH—, $C_1$—$C_4$-Dialkyl-N—, $C_1$—$C_4$-Alkoxy, $NO_2$, —CN, —COOH, —COO-Alkyl ($C_1$—$C_4$), $C_1$—$C_6$-Alkyl, Halogen, insbesondere Fluor, Chlor oder Brom, $C_1$—$C_4$-Alkylthio, —SH, $C_1$—$C_4$-Alkylsulfonyl, —$SO_3H$, —$SO_2$—$NH_2$, —$SO_2$—NH-Alkyl ($C_1$—$C_4$) tragen können.

Der Alkylrest kann auch einen mono-, bi- oder tricyclischen Substituenten mit vorzugsweise 3 bis 10 Kohlenstoffatomen tragen, der seinerseits durch Hydroxy, Amino, Halogen, insbesondere Fluor, Chlor, Brom, oder —COOH substituiert sein kann.

Der Alkylrest trägt bevorzugt Substituenten wie Hydroxy, Alkoxy mit 1 bis 4 Kohlenstoffatomen, Mercapto, Alkylthio mit 1 bis 4 Kohlenstoffatomen, Halogen, Nitro, Amino, Monoalkylamino mit 1 bis 4 C-Atomen und Acylamino, wobei der Acylrest von aliphatischen Carbonsäuren mit 1 bis 6 C-Atomen abgeleitet ist.

Für die cyclischen mono-, bi- oder tricyclischen Reste $R_1$, R′ und R″ kommen die für die Alkylreste genannten Substituenten in Betracht.

Die Arylreste R′ und R″ können einen oder mehrere, vorzugsweise 1 bis 3 gleiche oder verschiedene Substituenten tragen. Als Substituenten seien beispielhaft aufgeführt: Alkyl mit 1 bis 10 C-Atomen, das seinerseits wieder beispielsweise durch Chlor, Nitro oder Cyan substituiert sein kann, gegebenenfalls substituierte Alkenylreste mit 1 bis 10 Kohlenstoffatomen; Hydroxy, Alkoxy mit vorzugsweise 1 bis 4 Kohlenstoffatomen; Amino, Monoalkyl- und Dialkylamino mit vorzugsweise 1 bis 4 Kohlenstoffatomen je Alkylrest; Mercapto, Alkylthio mit vorzugsweise 1 bis 4 Kohlenstoffatomen; Carboxy, Carbalkoxy mit vorzugsweise 1 bis 4 Kohlenstoffatomen, die Sulfonsäuregruppe, Alkylsulfonyl mit vorzugsweise 1 bis 4 Kohlenstoffatomen Arylsulfonyl, vorzugsweise Phenylsulfonyl; Aminosulfonyl-, Alkylamino- und Dialkylaminosulfonyl mit 1 bis 4 Kohlenstoffatomen je Alkylgruppe vorzugsweise Methyl- und Dimethylaminosulfonyl; Nitro, Cyan oder die Aldehydgruppe; Alkylcarbonylamino mit vorzugsweise 1 bis 4 Kohlenstoffatomen; Alkylcarbonyl mit 1 bis 4 Kohlenstoffatomen, Benzoyl, Benzylcarbonyl und Phenyläthylcarbonyl, wobei die zuletzt genannten Alkyl, Phenyl, Benzyl und Phenyläthylreste ihrerseits wieder beispielsweise durch Chlor, Nitro oder Hydroxy substituiert sein können.

Die heterocyclischen Reste R′ und R″ sind bevorzugt von heteroparaffinischen, heteroaromatischen oder heteroolefinischen 5- oder 6-gliedrigen Ringen mit vorzugsweise 1 bis 3 gleichen oder verschiedenen Heteroatomen abgeleitet. Als Heteroatome stehen Sauerstoff, Schwefel oder Stickstoff. Diese Ringsysteme können weitere Substituenten wie beispielsweise Hydroxy-, Amino- oder $C_1$—$C_4$-Alkylgruppen tragen oder an sie können Benzolkerne oder weitere vorzugsweise 6-gliedrige heterocyclische Ringe der genannten Art annelliert sein.

Besonders bevorzugte heterocyclische Reste leiten sich beispielsweise von Furan, Pyran, Pyrrolidin, Piperidin, Pyrazol, Imidazol, Pyrimidin, Pyridazin, Pyrazin, Triazin, Pyrrol, Pyridin, Benzimidazol, Chinolin, Isochinolin oder Purin ab.

In den Verbindungen der Formel I steht $R_2$ vorzugsweise für —H, —OH, —$SO_3H$, —CN, —$CH_2NH_2$,

$$-CH_2NH-(C_1-C_{14}\text{-Alkyl}),\ -CH_2NH-\underset{\underset{O}{\|}}{C}-(C_1-C_{14}\text{-Alkyl}),$$

$$-CH_2-NH-SO_2-(C_1\text{ bis }C_{14})\text{-Alkyl}\ -CH_2-NH-SO_2\text{-Phenyl},$$

$$R'-NH-\underset{\underset{O}{\|}}{C}-NH-CH_2-,\ R'-NH-\underset{\underset{S}{\|}}{C}-NH-CH_2-\text{ oder }R'-O-\underset{\underset{O}{\|}}{C}-NH-CH_2-.$$

Ganz besonders bevorzugt steht $R_2$ für —H, —$SO_3H$, —CN,

$$R'-NH-\underset{\underset{O}{\|}}{C}-NH-CH_2-.$$

$R_3$ steht bevorzugt für Wasserstoff, $—CH_2OH$, $—CH_3$, $—CH_2NH_2$, $—CH_2NH—(C_1—C_6\text{-Alkyl})$ oder

$$—CH_2NH—\underset{\underset{O}{\|}}{C}—(C_1—C_6\text{-Alkyl})$$

oder $—CH_2—O—(C_1—C_6\text{-Alkyl})$.
Ganz besonders bevorzugt jedoch steht $R_3$ für $—CH_2OH$.

Es wurde gefunden, daß die neuen Verbindungen der Formel I potente Inhibitoren für α-Glucosidasen, insbesondere für Disaccharidasen sind. Daher sind die neuen Verbindungen wertvolle Mittel zur Beeinflussung einer Vielzahl von Stoffwechselvorgängen und bereichern somit den Arzneimittelschatz. Gegenüber dem aus der DT—OS 2 656 602 bekannten 2-Hydroxymethyl-3,4,5-trihydroxypiperidin weisen die neuen Verbindungen vorteilhafte therapeutische Eigenschaften auf.

Insbesondere zeigt sich bei der Fütterung von Ratten mit gekochter Stärke, daß die Zugabe einer erfindungsgemäßen Verbindung verglichen mit der vorgenannten Verbindung des Standes der Technik den Anstieg des Blutglucosespiegels wesentlich stärker hemmt.

Im folgenden wird anstelle der sterischen Formel I aus schreibtechnischen Gründen eine planare Schreibweise gewählt, unter der, soweit es nicht durch die jeweilige Formel bestimmt ist, die sterische Anordnung gemäß Formel I zu verstehen ist.

Weiterhin wurde gefunden, daß man Verbindungen der Formel I erhält, wenn man in Verbindungen der Formel II oder IIa,

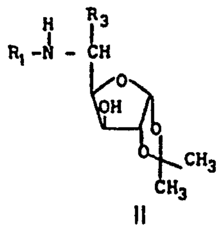

II

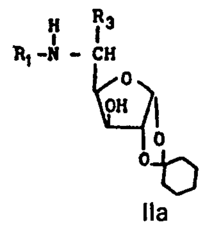

IIa

in der
$R_1$ und $R_3$ die oben angegebene Bedeutung haben,
durch vorsichtige Säurehydrolyse die Isopropyliden- oder Cyclohexylidenschutzgruppe entfernt, wobei es gegebenenfalls zweckmäßig ist, die Verbindungen der Formel I in der Form von Addukten der schwefligen Säure oder der Blausäure abzufangen ($R_2 = SO_3H$ oder CN). Aus dem Bisulfitadditionsprodukten werden die Verbindungen der Formel I mit $R_2 = OH$ durch Behandlung mit Basen, vorzugsweise Erdalkalihydroxiden wie $Ca(OH)_2$, oder $Sr(OH)_2$, insbesondere aber $Ba(OH)_2$ in Freiheit gesetzt. Durch Umsetzung mit Wasserstoff-Donor-Reduktionsmitteln wie beispielsweise $NaBH_4$ werden aus den Verbindungen der Formel I mit $R_2 = OH$ die Verbindungen der Formel I mit $R_2 = H$ gewonnen.

Weiterhin wurde gefunden, daß man Verbindungen der Formel I erhält, wenn man die Verbindungen der Formel I mit $R_2$ = OH in an sich bekannter Weise mit Blausäure zu Verbindungen der Formel mit $R_2$ = CN umsetzt und gegebenenfalls aus diesen durch katalytische Hydrierung der Nitrilgruppe Verbindungen mit $R_2 = —CH_2NH_2$ erhält und die Aminogruppe gegebenenfalls in an sich bekannter Weise zu Verbindungen, bei denen $R_2 = R'CONCH_2—$, $R'CONR''CH_2—$, $NHR'—CH_2—$, $NR'R''—CH_2—$ oder $R'SO_2NHCH_2—$ ist, acyliert, alkyliert oder sulfonyliert.

Die Verbindungen der Formel I, bei denen $R_2$, $—OR'$, $—SH$, $—SR'$, $—NH_2$, $—NHR'$ oder $—NR'R''$ ist, können erhalten werden, indem man Verbindungen der Formel I mit $R_2 = —OH$ in an sich bekannter Weise mit Alkoholen (R'OH), $H_2S$, Mercaptanen (R'SH), Ammoniak oder Aminen ($H_2NR'$, $HNR'R''$) umsetzt.

Die Verbindungen der Formel I, bei denen $R_2$ —COOH ist, werden erhalten, indem man Verbindungen der Formel I mit $R_2 = —CN$ in an sich bekannter Weise hydrolysiert.

Aus den so erhaltenen Carbonsäuren lassen sich in an sich bekannter Weise Verbindungen der Formel I mit $R_2 = —COOR'$ durch Umsetzung mit Alkoholen (R'OH), Verbindungen der Formel I mit $R_2 = —CONHR'$ oder $—CONR'R''$ oder $—CONH_2$ durch Aminolyse der Ester mit $NH_3$, $R'NH_2$ bzw. $R'R''N$ erhalten.

Verbindungen der Formel I mit $R_2 = —OH$ können auch erhalten werden, wenn man Verbindungen der Formel II in einem Reaktionsschritt A mit Trifluoracetanhydrid zu Verbindungen der Formel III umsetzt und dann im Reaktionsschritt B durch Säurehydrolyse die Isopropylidenschutzgruppe abspaltet und anschließend im Schritt C im neutralen bis alkalischen Reaktionsmedium die Trifluoracetylgruppe der Verbindung IV entfernt.

Die angegebene Reaktionsfolge läßt sich wie folgt veranschaulichen:

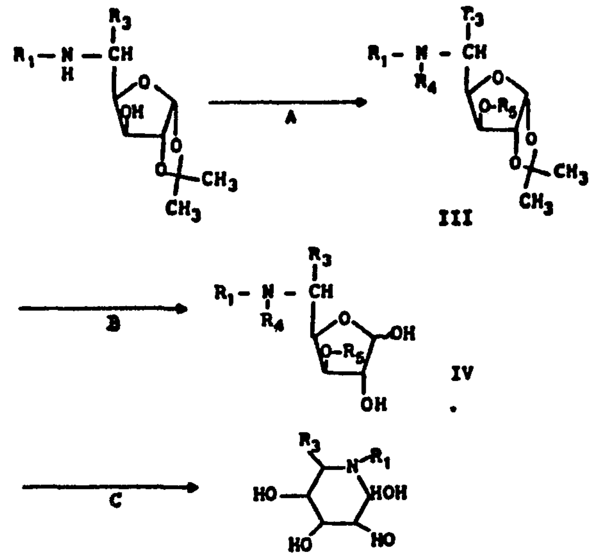


III

IV

In den Formeln stehen
$R_4$ für Trifluoracetyl und
$R_5$ für Trifluoracetyl oder Wasserstoff.

Diese Reaktionsfolge läßt sich analog auf Verbindungen der Formel IIa übertragen.

Es wurde auch gefunden, daß man Verbindungen der Formel I mit $R_2 = H$ erhält, wenn man Verbindungen der Formel V

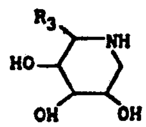

V

mit Carbonylverbindungen der Formel VI

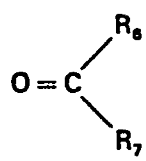

VI

in der $R_6$ und $R_7$ entweder H oder die für $R_1$ gegebene Bedeutung haben oder Glieder eines alicyclischen oder heterocyclischen Ringes sind, in Gegenwart eines Wasserstoff-Donor-Reduktionsmittels umsetzt.

Ferner erhält man Verbindungen der Formel I mit $R_2 = H$, wenn man Amide der Formel VII

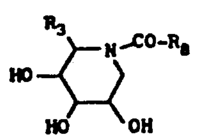

VII

in der $R_8$ entweder H ist oder die für $R_1$ gegebene Bedeutung hat, oder Carbamate der Formel VIII

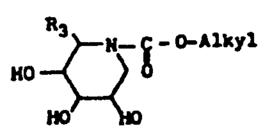

VIII

— gegebenenfalls auch mit Hydroxyschutzgruppen versehene Derivate dieser Verbindungen — mit einem Amid-Reduktionsmittel zu Aminen reduziert.

Ein weiteres Verfahren zur Herstellung von Verbindungen der Formel I mit $R_2 = H$ besteht darin, daß man Verbindungen der Formel V mit reaktiven Alkylierungsmitteln der Formel IX

$$Z—R_1 \qquad IX$$

umsetzt, wobei $R_1$ die oben für Alkyl angegebene Bedeutung hat und Z eine in Alkylierungsmitteln gebräuchliche leicht austretende Gruppe wie beispielsweise Halogenid oder $^{\ominus}O—SO_3H$ ist.

Bei den genannten Verfahren gilt jeweils, daß, wenn $R_3 = CH_2OH$ ist, die Reste $R_1$ in Z—$R_1$ sowie $R_6$, $R_7$ und $R_8$ so gewählt werden, daß im Reaktionsprodukt $R_1$ nicht $C_1$—$C_4$-Alkyl darstellt.

Des weiteren erhält man Verbindungen der Formel I, wenn man beispielsweise in Verbindungen der Formel I mit $R_3 =$ —$CH_2OH$ die —$CH_2OH$-Gruppe selektiv in an sich bekannter Weise in eine

—$CH_2$—O—$SO_2$ 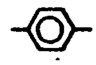 $CH_3$-Gruppe

verwandelt und diese entweder reduktiv in die —$CH_3$-Gruppe oder über eine —$CH_2$—$N_3$-Gruppe reduktiv in eine Aminogruppe überführt. Verbindungen der Formel I erhält man auch, wenn man in Verbindungen der Formel I mit $R_3 =$ —$CH_2NH_2$ die Aminogruppe in an sich bekannter Weise mit Aldehyden oder Ketonen in Gegenwart eines Wasserstoffdonors, mit Alkylhalogeniden, Carbonsäure- oder Sulfonsäurechloriden, Chlorkohlensäureestern, Isocyanaten und Senfölen derivatisiert.

Verbindungen der Formel I, in denen $R_1$ ein durch eine Acylamino-, Sulfonylamino-, Alkoxycarbonylamino-, Ureido-oder eine Thioureidogruppe substituierter aliphatischer oder aromatischer Rest ist, erhält man ausgehend von Verbindungen der Formel I, in denen $R_1$ ein durch eine Aminogruppe substituierter aliphatischer oder aromatischer Rest ist, durch Umsetzung dieser Aminogruppe mit Carbonsäure- oder Sulfonsäurechloriden, mit Chlorkohlensäureestern, Isocyanaten oder Senfölen in an sich bekannter Weise.

Die einzelnen Verfahrensweisen zur Herstellung der erfindungsgemäßen Wirkstoffe werden im folgenden veranschaulicht:

Verwendet man als Ausgangsstoff eine Verbindung der Formel II mit $R_1$ = Aethyl (nur beispielhaft, hier nicht beansprucht), so läßt sich der Reaktionsablauf wie folgt wiedergeben:

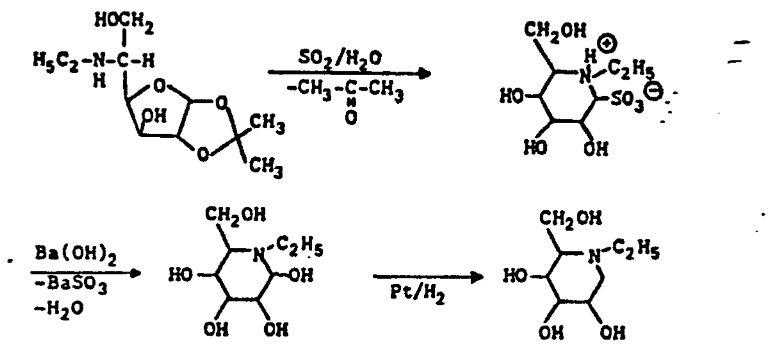


Mit 1-Desoxynojirimycin der Formel V und Formaldehyd (beispielhaft; hier nicht beansprucht) als Ausgangsstoffe ergibt sich

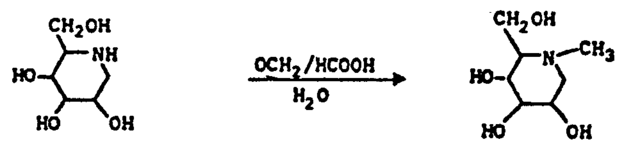


Mit Benzaldehyd als Carbonylkompoennte wird die reduktive Alkylierung wie folgt durchgeführt:

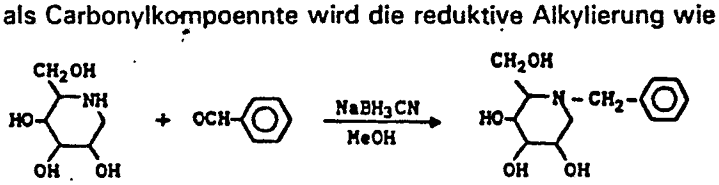


Geht man von Säureamiden der Formel VII aus, so läßt sich die Reaktion wie folgt beschreiben:

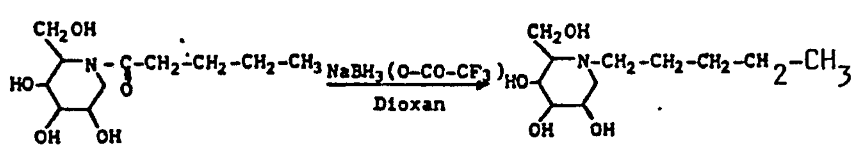


Urethane der Formel VIII — gegebenenfalls als mit Hydroxylschutzgruppen versehene Derivate — lassen sich mit $LiAlH_4$ zum n-Methyl-1-desoxynojirimycin (beispielhaft, hier nicht beansprucht) reduzieren:

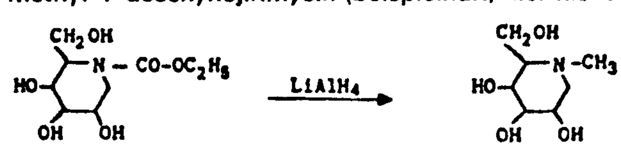

Für die Reaktion von 1-Desoxynojirimycin mit Alkylierungsmitteln sei die Reaktion mit Allylbromid als Beispiel angegeben:

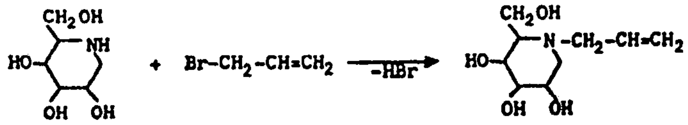


Die als Ausgangsstoffe verwendeten Verbindungen der Formel II sind zum Teil bekannt. Dies ist der Fall, wenn $R_3 = H$, $—CH_2OH$ oder $—CH_2NH_2$ und $R_1 = H$ ist. Andere Verbindungen der Formel II bzw. II a sind neu; sie können aber nach an sich bekannten Verfahren aus literaturbekannten Verbindungen hergestellt werden.

So kann man beispielsweise von der literaturbekannten Verbindung der Formel X

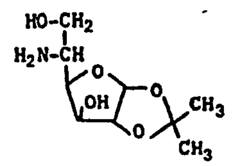

X

ausgehen und diese mit Carbonylverbindungen der Formel VI in Gegenwart eines Wasserstoff-Donor-Reduktionsmittels zu Verbindungen der Formel II umsetzen.

Des weiteren kann man die Verbindung X mit reaktiven Säurederivaten zu Säureamiden oder Urethanen umsetzen und diese mit einem Amid-Reduktionsmittel zu Aminen reduzieren. Dies sei an einem Beispiel veranschaulicht:

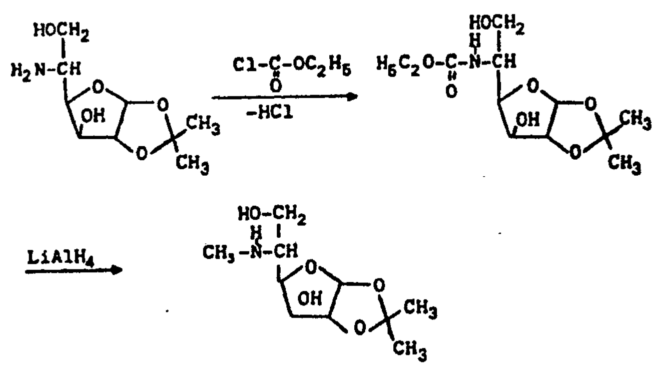


Die Verbindung der Formel X kann man auch mit reaktiven Alkylierungsmitteln der Formel IX

$$Z—R_1 \qquad IX$$

zu Verbindungen der Formel II umsetzen.

Des weiteren kann man auch in die oben erwähnten Reaktionen anstelle der Verbindung X bekannte partiell geschützte Derivate der Formel XI einsetzen.

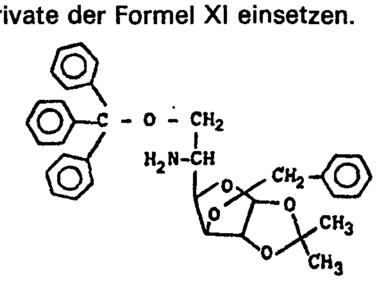

XI

und anschließend die Trityl- und Benzyl-Schutzgruppen auf bekanntem Wege, beispielsweise mit Natrium in flüssigem Ammoniak, entfernen. Zur Darstellung von Verbindungen der Formel II kann auch die ebenfalls literaturbekannte Verbindung der Formel XII

Tr ≡ 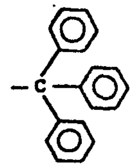

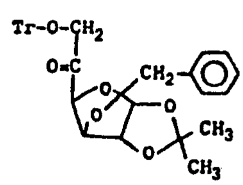

XII

mit Aminen der Formel XIII

$$R_1—NH_2 \qquad XIII$$

in Gegenwart eines Wasserstoff-Donor-Reduktionsmittels, beispielsweise in Gegenwart von $NaBH_3CN$, umgesetzt werden. Bei dieser Reaktion entsteht in der Regel ein Diastereomerengemisch. Das nicht erwünschte Diastereomere wird gegebenenfalls auf dieser Stufe oder auf einer späteren Stufe durch die üblichen chromatographischen Methoden oder durch fraktionierte Kristallisation abgetrennt. Schließlich werden die Trityl-und Benzylschutzgruppe auf bekanntem Wege, beispielsweise mit Natrium, in flüssigem Ammoniak abgespalten.

Des weiteren kann man neue Verbindungen der Formel II bzw. IIa auch erhalten, indem man die literaturbekannten Abbauprodukte der D-Glucose der Formeln XIV bis XVI

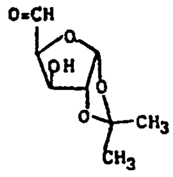
XIV
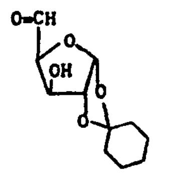
XV
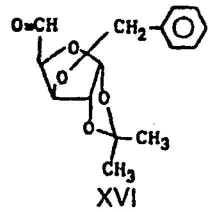
XVI

mit Reagentien mit Carbanioncharakter wie beispielsweise Alkyl-Li oder Grignard-Verbindungen oder dem Li-Salz des 1,3-Dithians zur Reaktion bringt und die erhaltenen Verbindungen der Formel XVII

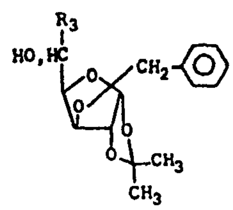
XVII

in an sich bekannter Weise [S. INOUYE et al., Tetrahedron *23*, 2125—2144] über das Keton und das Oxim in das Amin umwandelt, wobei in der Regel ein Gemisch von gluco- und ido-Verbindung entsteht, aus dem sich die gewünschte gluco-Verbindung XVIII durch die üblichen chromatographischen Methoden isolieren läßt.

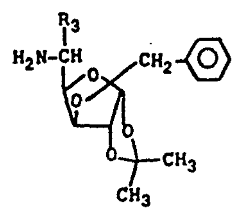
XVIII

Die Entfernung der Benzylschutzgruppe durch katalytische Hydrierung oder mit Na in flüssigem $NH_3$ liefert dann die Verbindungen der Formel II.

Verbindungen der Formel XIX erhält man, wenn man die Aldehyde der Formeln XIV bis XVI in an sich bekannter Weise mit Aminen und Blausäure zu Aminonitrilen umsetzt, beispielsweise XVI zu XIX

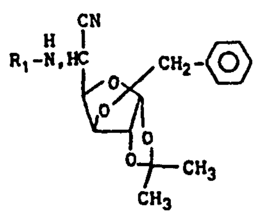
XIX

wobei auch hier in der Regel die gewünschte gluco-Verbindung von der ido-Verbindung durch übliche chromatographische Methoden abgetrennt werden muß. Die weitere Abwandlung der Nitrilgruppe durch Hydrierung oder Hydrolyse von oder nach der Entfernung der Benzylschutzgruppe führt zu weiteren Verbindungen der Formel II.

Die Umsetzung von XIV bis XVI mit CH-aciden Verbindungen wie beispielsweise Nitroalkanen, Alkylnitrilen, CH-aciden Estern oder Ketonen kann ebenfalls zu Verbindungen der Formel II führen. Dabei erhält man entweder direkt oder durch Dehydratisierung der Aldoladditionsprodukte ungesättigte Verbindungen beispielsweise der Formel XX,

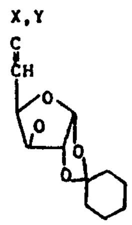

X = —$NO_2$, —CN, —COOAlkyl
Y = H, Alkyl, Aryl

XX

die durch Michael-Addition von Aminen nach chromatographischer Trennung von gluco- und ido-Isomeren Verbindungen der Formel IIa liefern.

Die Abspaltung der Isopropylidenschutzgruppe aus den Verbindungen der Formel II erfolgt in mäßig stark saurer bis schwach saurer Lösung, bevorzugt in einem pH-Bereich zwischen 1 und 4, in wäßriger Lösung oder in einem mit Wasser mischbaren, wasserhaltigen organischen Lösungsmittel. Als Säuren können verdünnte Mineralsäuren wie beispielsweise Schwefelsäure oder auch organische Säuren wie Essigsäure verwendet werden. Die Reaktion wird bevorzugt bei Atmosphärendruck und einer Temperatur zwischen Raumtemperatur und der Siedetemperatur des Lösungsmittels durchgeführt.

Zur Aufarbeitung des Reaktionsansatzes wird die Säure neutralisiert und als Salz oder mit Hilfe eines basischen Ionenaustauschers abgetrennt. Die Isolierung der Verbindungen der Formel I mit $R_2 = OH$ erfolgt dann gegebenenfalls durch ein schonendes Entfernen des Lösungsmittels, beispielsweise durch Lyophilisation.

Eine bevorzugte Ausführungsform der Abspaltung der Isopropylidenschutzgruppe aus Verbindungen der Formel II besteht darin, daß man die wäßrige oder Wasserhaltige alkoholische Lösung der Verbindungen der Formel II mit $SC_2$ sättigt und mehrere Tage bei Temperaturen zwischen 20° und 50°C aufbewahrt. Die Verbindungen der Formel I fallen dann als meist gut kristallisierende Bisulfitaddukte ($R_2 = —SO_3H$) an, aus denen sich die Verbindungen der Formel I mit Hilfe von z.B. wäßrigem $Ba(OH)_2$ freisetzen lassen.

Die Reduktion von Verbindungen der Formel I mit $R_2 = OH$ zu Verbindungen der Formel I mit $R_2 = H$ erfolgt durch Verwendung von Alkalimetallborhydriden, Alkalimetallcyanoborhydriden oder auch von Dialkylaminoboranen. Bevorzugt ist die Verwendung von Natriumborhydrid in wäßriger Lösung oder in einem mit Wasser mischbaren wasserhaltigen organischen Lösungsmittel, wie beispielsweise Dioxan, bei Raumtemperatur oder gegebenenfalls erhöhter Temperatur. Ganz besonders bevorzugt erfolgt die Reduktion jedoch katalytisch mit Pt oder Pd als Katalysator oder in Gegenwart von Raney-Ni. Dabei arbeitet man bevorzugt in wäßriger Lösung bei Raumtemperatur.

Verbindungen der Formel I werden weiterhin dadurch erhalten, daß man Verbindungen der Formel

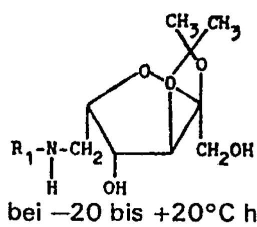

mit starker Mineralsäure vom pH <1 bei −20 bis +20°C hydrolysiert und anschließend bei pH 4 bis 6 mit z.B. $H_2$/Raney-Nickel, $H_2/P + O_2$ oder $NaBH_4$ hydriert.

Die Verbindungen der Formel XXI werden erhalten, indem man Verbindungen der Formel

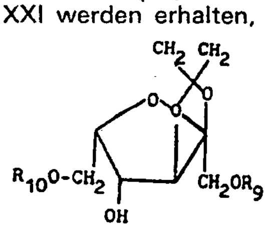

worin $R_9$ H oder $CH_3CO$ und $R_{10}$ Mesyl oder Tosyl bedeuten mit Aminen der Formel

$$R_1—NH_2$$

bei 20 bis 150°C in einem polaren Lösungsmittel, z.B. einem Alkohol, Dimethylsulfozid oder in überschüssigem Amin umsetzt.

Das Ausgangsprodukt der Formel V mit $R_3 = —CH_2OH$ ist bekannt und wird entweder durch katalytische Hydrierung aus dem durch Fermentation erhältlichen Nojirimycin [s. S. INOUYE et al., Tetrahedron *23*, 2125—2144 (1968)] oder durch Extraktion aus Maulbeerbaumrinde (s. DT—OS 2 656 602) oder aber vollsynthetisch gewonnen. Nach einem neuen vorteilhaften Verfahren kann man 1-Desoxynojirimycin auch dadurch herstellen, daß man Organismen der Familie Bacillaceae in üblichen

Nährlösungen bei Temperaturen von etwa 15 bis etwa 80°C etwa 1 bis etwa 8 Tage unter Belüftung in üblichen Fermentationsgefäßen kultiviert, die Zellen abschleudert und die Desoxyverbindung aus der Kulturbrühe oder den Zellextrakten durch übliche Reinigungsverfahren isoliert [Deutsche Offenlegungsschrift 26 58 563.7].

Die Carbonylverbindungen der Formel VI sind entweder bekannt oder können nach Standardverfahren hergestellt werden.

Als typische Beispiele seien im einzelnen genannt:

Gerad- oder verzweigtkettige Alkylaldehyde wie Formaldehyd, Acetaldehyd, n-Propanal, n-Butanal, 2-Methylpropanal, n-Pentanal, 2-Methylbutanal, 3-Methylbutanal, 2,2-Dimethylpropanal, n-Hexanal, 2-Äthylbutanal, n-Heptanal und n-Octanal; Alkenylaldehyde wie Propenal, 2-Methylpropenal, 2-Butenal, 2-Methyl-2-butenal, 2-Äthyl-2-hexenal; Cyclische Aldehyds wie Cyclopropancarbaldehyd, Cyclopentancarbaldehyd, Cyclopentancetaldehyd, Cyclohexancarbaldehyd; Benzaldehyd, o-, m- und p-toluolcarbaldehyde und Phenylacetaldehyd; durch Hydroxy substituierte gerad- und verzweigtkettige Alkylaldehyde wie 5-Hydroxypentanal, 2-Hydroxy-3-methylbutanal, 2-Hydroxy-2-methylpropanal, 4-Hydroxybutanal, 2-Hydroxypropanal und 8-hydroxyoctanal; durch Amino substituierte gerad- und verzweigtkettige Alkylaldehyde wie 5-Aminopentanal, 2-Aminopropanal, 3-Aminopropanal, 4-Aminobutanal, 2-Amino-3-methylbutanal, 8-Aminooctanal and mono-N-Alkylderivate davon; und durch Amino und Hydroxy disubstituierte gerad- und verzweigtkettige Alkylaldehyde wie 2-Hydroxy-5-aminopentanal, 3-Hydroxy-3-methyl-4-aminobutanal, 2-Hydroxy-4-aminobutanal, 2-Hydroxy-3-aminopropanal, 2-Hydroxy-2-methyl-3-aminopropanal, 2-Amino-3-hydroxyoctanal und mono-N-Alkylderivate davon.

Des weiteren:

Methoxy-acetaldehyd, Aethoxy-acetaldehyd, n-Propoxy-acetaldehyd, i-Propoxy-acetaldehyd, n-Butoxy-acetaldehyd, i-Butoxy-acetaldehyd, tert.-Butoxy-acetaldehyd, Cyclopropylmethyloxy-acetaldehyd, Cyclopropoxyacetaldehyd, 2-Methoxy-äthoxy-acetaldehyd, 2-Aethoxy-äthoxy-acetaldehyd, 2-Methoxy(1-methyl-äthoxy)-acetaldehyd, 2-Aethoxy(1-methyl-äthoxy)-acetaldehyd, Phenyloxy-acetaldehyd, 2-Methoxy-2-methyl-acetaldehyd, 2-Aethoxy-2-methyl-acetaldehyd, 2-n-Propoxy-2-methyl-acetaldehyd, 2-(i-Propoxy-) 2-methyl-acetaldehyd, 2-(n-Butoxy)-2-methyl-acetaldehyd, 2-(i-Butoxy)-2-methyl-acetaldehyd, 2-(tert.-Butoxy)-2-methyl-acetaldehyd, 2-Cyclopropylmethyloxy-2-methyl-acetaldehyd, 2-Cyclopropyloxy-2-methyl-acetaldehyd, 2-Methoxy-äthoxy-$\alpha$-methyl-acetaldehyd, 2-Aethoxy-äthoxy-$\alpha$-methyl-acetaldehyd, 2-Methoxy-(1-methyl-äthoxy)$\alpha$-methyl-acetaldehyd, 2-Methoxy-2,2-dimethyl-acetaldehyd, 2-Aethoxy-2,2-dimethyl-acetaldehyd, 2-Cyclopropylmethyloxy-acetaldehyd, 2-$\omega$-Butoxy-2,2-dimethyl-acetaldehyd, Methylthio-acetaldehyd, Aethylthio-acetaldehyd, n-Propylthio-acetaldehyd, i-Propylthio-acetaldehyd, Cyclopropylmethylthioacetaldehyd, 3-Methoxy-propanol, 3-Aethoxypropanal, 3-n- und 3-i-propoxy-propanal, 3-n-, 3-i- und 3-tert.-Butoxy-propanal, 3-Cyclopropyloxy-propanal, 3-Cyclopropylmethyloxy-propanal, 3-Methoxy-3-methyl-propanal, 3-Aethoxy-3-methyl-propanal, 3-n- und 3-i-propoxy-3-methyl-propanal, 3-n-, 3-i- und 3-tert.-Butoxy-3-methyl-propanal, 2,3 und 4-Methoxy-butanal, 2,3 und 4-Aethoxy-butanal, 2-Methylthio-propanal, 2-Aethylthio-propanal, 3-Methylthio-propanal, 3-Aethylthio-propanal, 2-Methylthio-butanal, 3-Methylthio-butanal, 4-Methylthio-butanal, Furfurol, Tetrahydrofurfurol, Thiophen, 5-Bromthiophen, 5-Methylfurfurol, Pyran-carbaldehyd.

Außerdem seien als Ketone beispielsweise genannt:

Aceton, Methyläthylketon, Methyl-n-propylketon, Diäthylketon, Methylbutylketon, Cyclopentanon, Di-n-propyl-keton, Cyclohexanon, 3-Methylcyclohexanon, 4-Methylcyclohexanon, Acetophenon, Propiophenon, Butyrophenon, Phenylaceton, p-Methoxyacetophenon, m-Nitroacetophenon.

Als Wasserstoff-Donor-Reduktionsmittel kann man beispielsweise Ameisensäure verwenden (Leuckart-Wallach-Reaktion). Die Ameisensäure wird in großem Ueberschuß verwendet. Mit Formaldehyd als Carbonylkomponente kann die Reaktion in wäßriger Lösung durchgeführt werden, mit Ketonen und weniger reaktionsfähigen Aldehyden in wasserfreier Ameisensäure. Die Reaktionstemperaturen liegen zwischen 100 und 200°C, gegebenenfalls muß die Reaktion in einem Autoklaven durchgeführt werden.

Als Wasserstoff-Donor-Reduktionsmittel kann man auch katalytisch erregten Wasserstoff verwenden. Als Katalysator kommt vor allem Raney-Nickel in Frage, es können aber auch Edelmetallkatalysatoren Verwendung finden. Die Reaktion wird im allgemeinen bei Drucken zwischen 80 und 150 Atmosphären $H_2$-Druck und Temperaturen zwischen 70 und 150°C durchgeführt. Als Lösungsmittel werden protische, polare Lösungsmittel besonders Alkohole bevorzugt.

Als Wasserstoff-Donor-Reduktionsmittel werden auch Alkalimetallcyanoborhydride, Dialkylaminoborane und Alkalimetallborhydride verwendet. Besonders bevorzugt in dieser Verfahrensvariante ist die Verwendung von Natriumcyanoborhydrid.

Die Reaktion wird im allgemeinen bei Raumtemperatur durchgeführt. Es kann aber auch günstig sein, auf Rückflußtemperatur zu erhitzen.

Das Verfahren wird üblicherweise in einem inerten Lösungsmittel durchgeführt. Obwohl wasserfreie aprotische Lösungsmittel eingesetzt werden können (z.B. Tetrahydrofuran, wenn das Reduktionsmittel Morpholinoboran ist), wird gewöhnlich doch ein protisches Lösungsmittel verwendet. Als solches eignet sich besonders ein niederes Alkanol. Es kann aber auch Wasser oder ein wäßriges

niedriges Alkanol (z. B. wäßriges Methanol) oder andere wäßrige Lösungsmittelsysteme, wie z. B. wäßriges Dimethylformamid, wäßriges Hexamethylphosphorsäuretriamid, wäßriges Tetrahydrofuran oder wäßriger Aethylenglycoldimethyläther verwendet werden.

Das Verfahren wird gewöhnlich in einem pH-Bereich von 1 bis 11 durchgeführt, bevorzugt ist ein pH-Bereich zwischen 4 und 7.

Die Säureamide der Formel VII und Urethane der Formel VIII sind zum Teil bekannt oder können nach bekannten Verfahren aus Verbindung V und reaktiven Säurederivaten, die auch in situ aus den freien Säuren gebildet werden können, erhalten werden.

Dabei kann die Reaktion so geführt werden, daß nur die Aminogruppe der Verbindung V mit dem Säurederivat reagiert, beispielsweise durch Verwendung überschüssigen Säureanhydrids in einer wäßrigen oder alkoholischen Lösung oder aber so, daß zunächst die peracylierten Verbindungen entstehen, die dann durch Umsetzung mit alkoholischem Ammoniak oder durch Alkalialkoholat katalysiert Umesterung in die N-acylierten Verbindungen überführt werden. Letzteres Verfahren sei an einem Beispiel erläutert:

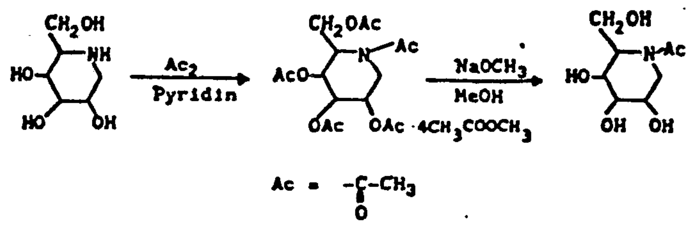


Die Reduktion der Säureamide der Formel II zu Aminen der Formel I (R = H) kann mit komplexen Metallhydriden oder auch mit Borwasserstoffverbindungen erfolgen. Bevorzugt ist die Verwendung von $NaBH_4$ in Pyridin oder auch von Natriumacyloxyborhydriden, besonders die von Natriumtrifluoracetoxyborhydrid. Die Reduktionsmittel werden in der Regel im Ueberschuß eingesetzt. Natriumtrifluoracetoxyborhydrid wird in situ aus Natriumborhydrid und Trifluoressigsäure erzeugt. Als Lösungsmittel kommen neben Pyridin polare aprotische Lösungsmittel wie Dioxan, Tetrahydrofuran oder Diglyme in Frage. Die Reaktion wird bevorzugt bei der Siedetemperatur des Lösungsmittels durchgeführt. Gegebenenfalls kann auch $LiAlH_4$ zur Reduktion verwendet werden, bevorzugt dann, wenn die Hydroxylgruppen vorher auf üblichem Wege geschützt werden.

Die reaktiven Alkylierungsmittel der Formel IX sind bekannt oder können nach gängigen Verfahren hergestellt werden. Die Umsetzung mit der Verbindung V erfolgt in inerten organischen Lösungsmitteln bei Raum- bis Siedetemperatur mit oder ohne Zusatz eines säurebindenden Mittels.

Als neue Wirkstoffe seien im einzelnen aufgeführt:

Verbindungen der Formel 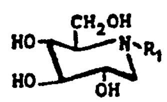

| $R_1$ |
|---|
| $CH_3(CH_2)_3$—$CH_2$— |
| 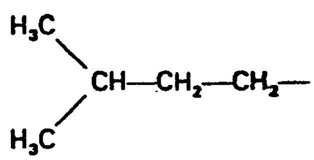 |
| 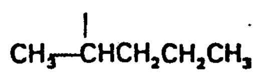 |
| 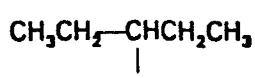 |

| $R_1$ |
|---|
| 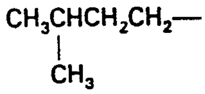 |
| $CH_3(CH_2)_4—CH_2—$ |
| $CH_3(CH_2)_5—CH_2—$ |
| 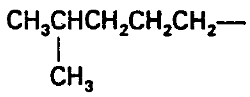 |
| 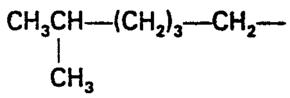 |
| $CH_3(CH_2)_6—CH_2—$ |
| 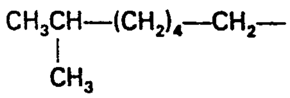 |
| $CH_3—(CH_2)_8—CH_2—$ |
| $CH_3—(CH_2)_{10}—CH_2—$ |
| $CH_3—(CH_2)_{12}—CH_2—$ |
| $CH_3(CH_2)_{14}—CH_2—$ |
| $CH_3—(CH_2)_{16}—CH_2—$ |
| 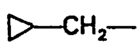 |
| 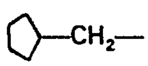 |
| 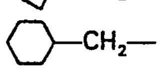 |
| 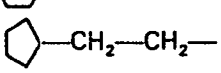 |
| $HO—CH_2—CH_2—$ |
| 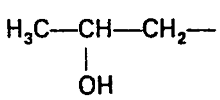 |
| $HOH_2C—CH_2—CH_2—$ |
| $HOH_2C—CH_2—CH_2—CH_2—$ |
| $HOH_2C—(CH_2)_3—CH_2—$ |
| 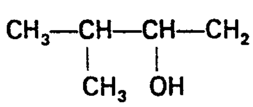 |
| 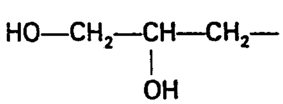 |
| $CH_3OCH_2—CH_2—$ |

| $R_1$ |
|---|
| $C_3H_7OCH_2—CH_2—$ |
| $CH_3COOCH_2—CH_2—$ |
| 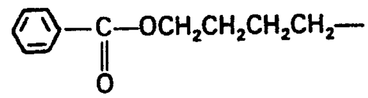 |
| $H_2N—CH_2—CH_2—$ |
| 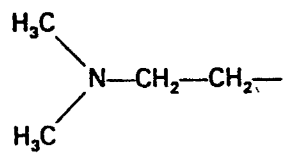 |
| $CH_3CONH—CH_2—CH_2$ |
| 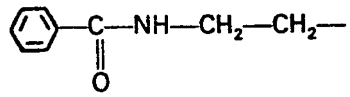 |
| 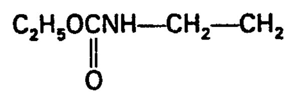 |
| 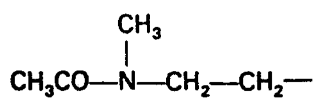 |
| $CH_3NH—CO—NH—CH_2CH_2—$ |
| 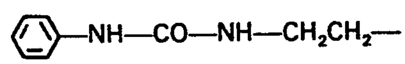 |
| $CH_3NH—CS—NH—CH_2CH_2—$ |
| 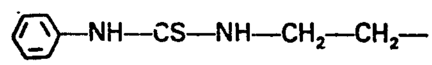 |
| $H_2N—CH_2CH_2CH_2—$ |
| $CH_3CONHCH_2CH_2CH_2—$ |
| 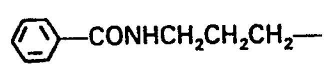 |
| $CH_3NHCONHCH_2CH_2CH_2—$ |
| $H_2N—CH_2CH_2CH_2CH_2—$ |
| $H_2C{=}CH—CH_2—$ |
| $H_3C—HC{=}CH—CH_2—$ |
| $H_2C{=}CH—CH_2—CH_2—$ |
| $H_2C{=}CH—CH_2—CH_2—CH_2—CH_2$ |
| $H_2C{=}CH—(CH_2)_7—CH_2—$ |
| $HOOC—CH_2—$ |
| $HOOC—CH_2—CH_2—$ |

$R_1$

$H_5C_2OOC-CH_2-CH_2-$

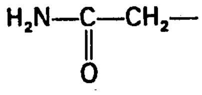

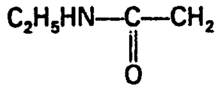

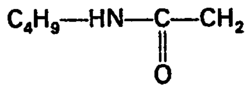

$HO_3S-CH_2CH_2CH_2-$

$H_2NO_2S-CH_2CH_2CH_2-$

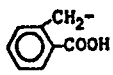

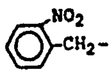

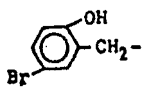

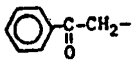

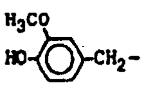

$H-C\equiv C-CH_2-$

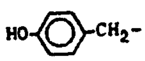

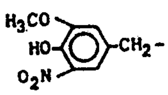

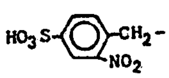

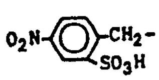

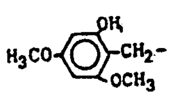

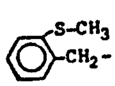

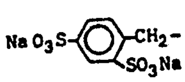

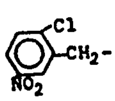

$R_1$

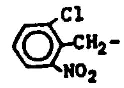

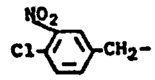

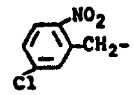

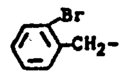

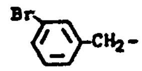

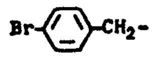

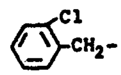

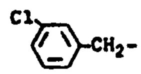

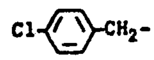

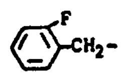

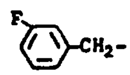

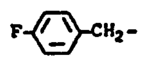

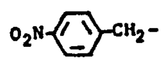

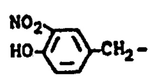

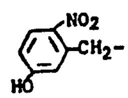

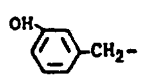

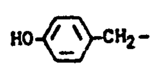

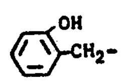

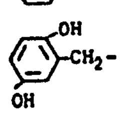

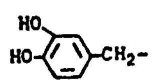

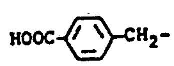

| $R_1$ | $R_1$ |
|---|---|
| 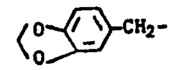 | 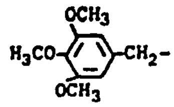 |
| 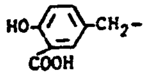 | 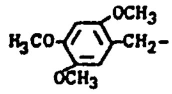 |
| 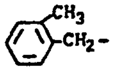 | 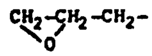 |
| 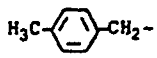 | 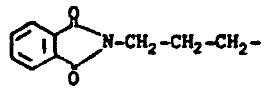 |
| 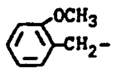 | 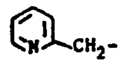 |
| 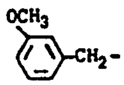 | 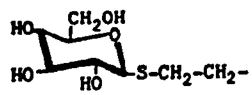 |
| 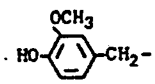 | 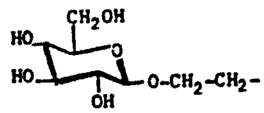 |
| 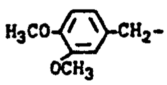 | 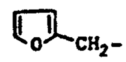 |
| 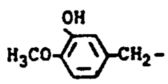 | 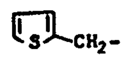 |
| 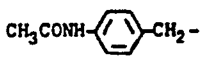 | 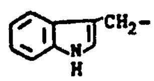 |
| 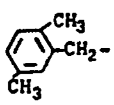 | 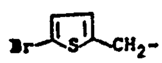 |
| 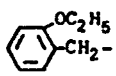 | 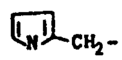 |
| 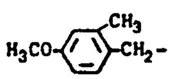 | 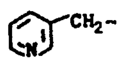 |
| 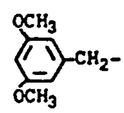 | 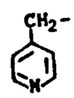 |
| 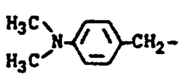 | 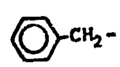 |

Verbindungen der Formel

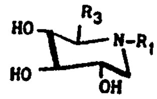

| $R_1$ | $R_3$ |
|---|---|
| H— | $CH_3$— |
| H— | $CH_3CH_2$— |
| H— | $CH_3CH_2CH_2$— |
| H— | $CH(CH_2)_6$—$CH_2$— |
| H— | $H_3C$—O—$CH_2$— |
| H— | $H_5C_2$—O—$CH_2$— |
| H— | $H_3C$—COO—$CH_2$— |
| H— | 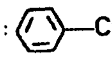—COO—$CH_2$— |
| H— | $H_2N$—$CH_2$— |
| H— | $CH_3CO$—NH—$CH_2$— |
| H— | 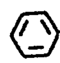—CO—NH—$CH_2$— |
| H— | 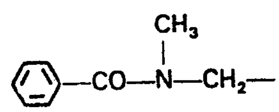 |
| H— | $CH_3NHCONH$—$CH_2$— |
| H— | 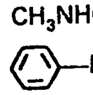—NHCONH—$CH_2$— |
| H— | $CH_3$—$CH_2$—NH—C(=S)—NH—$CH_2$ |
| H— | $C_2H_5OCONH$—$CH_2$— |
| H— | HO—$CH_2$—$CH_2$— |
| H— | —COOH |
| H— | —$CONH_2$ |
| H— | $H_3C$—$SO_2$—NH—$CH_2$— |
| H— | $H_3C$—$H_2C$—$SO_2$—NH—$CH_2$— |
| H | 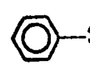—$SO_2$NH—$CH_2$— |
| $CH_3$ | $CH_3$— |
| $CH_3$— | $CH_3CH_2$— |
| $CH_3$— | $CH_3CH_2CH_2$— |

| $R_1$ | $R_3$ |
| --- | --- |
| $CH_3—$ | $CH_3(CH_2)_6—CH_2—$ |
| $CH_3—$ | $H_3C—O—CH_2—$ |
| $CH_3—$ | $H_5C_2—O—CH_2—$ |
| $CH_3—$ | $H_3C—COO—CH_2—$ |
| $CH_3—$ | 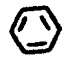 $—COO—CH_2—$ |
| $CH_3—$ | $H_2N—CH_2—$ |
| $CH_3—$ | $CH_3CO—NH—CH_2—$ |
| $CH_3—$ | 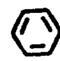 $—CO—NH—CH_2—$ |
| $CH_3—$ | 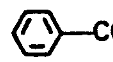 $—CO—\overset{CH_3}{\overset{\vert}{N}}—CH_2—$ |
| $CH_3—$ | $CH_3NHCONH—CH_2—$ |
| $CH_3—$ | 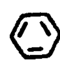 $—NHCONH—CH_2—$ |
| $CH_3—$ | $CH_3—CH_2—\underset{H}{N}—\underset{\underset{S}{\Vert}}{C}—NH—CH_2—$ |
| $CH_3—$ | $C_2H_5OCONH—CH_2—$ |
| $CH_3—$ | $HO—CH_2—CH_2—$ |
| $CH_3—$ | $—COOH$ |
| $CH_3—$ | $—CONH_2$ |
| $CH_3—$ | $H_3C—SO_2—\underset{H}{N}—CH_2—$ |
| $CH_3—$ | $H_3C—H_2C—H_2C—SO_2—\underset{H}{N}—CH_2—$ |
| $CH_3—$ | 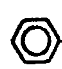 $—SO_2—\underset{H}{N}—CH_2—$ |

Verbindungen der Formel

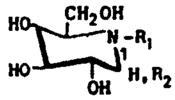

Die nachstehend aufgeführten Beispiele umfassen bezüglich der Konfiguration am C-1-Atom sowohl $\alpha$- als auch $\beta$-Form

| $R_1$ | $R_2$ |
|---|---|
| H— | $H_2N$—$CH_2$— |
| H— | $CH_3CO$—NH—$CH_2$— |
| H— | 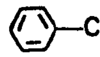—CO—NH—$CH_2$— |
| H— | 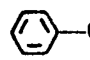—CO—N($CH_3$)—$CH_2$— |
| H— | $CH_3NHCONH$—$CH_2$— |
| H— | 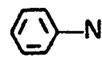—NHCONH—$CH_2$— |
| H— | $CH_3$—$CH_2$—NH—C(=S)—NH—$CH_2$— |
| H— | |
| H— | $C_2H_5OCONH$—$CH_2$— |
| H— | —COOH |
| H— | —$COOC_2H_5$— |
| H— | —$CONH_2$ |
| H— | $H_3C$—$SO_2$—NH—$CH_2$ |
| H | $H_3C$—$H_2C$—$H_2C$—$SO_2$—NH—$CH_2$— |
| H— | 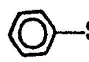—$SO_2$—NH—$CH_2$ |
| H— | HO—$CH_2$— |
| H— | $H_5C_2$—COO—$CH_2$— |
| $CH_3$— | $H_2N$—$CH_2$— |
| $CH_3$— | $CH_3CO$—$NHCH_2$— |
| $CH_3$— | 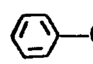—CO—NH—$CH_2$— |
| $CH_3$— | 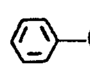—CO—N($CH_3$)—$CH_2$— |
| $CH_3$— | $CH_3NHCONH$—$CH_2$— |
| $CH_3$— | 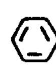—NHCONH—$CH_2$— |

| $R_1$ | $R_2$ |
|---|---|
| $CH_3—$ | $CH_3—CH_2—N(H)—C(=S)—NH—CH_2—$ |
| $CH_3—$ | $C_2H_5OCONH—CH_2—$ |
| $CH_3—$ | $—COOH$ |
| $CH_3—$ | $—COOC_2H_5$ |
| $CH_3—$ | $—CONH_2$ |
| $CH_3—$ | $H_3C—SO_2—N(H)—CH_2—$ |
| $CH_3—$ | $H_3C—H_2C—H_2C—SO_2—N(H)—CH_2—$ |
| $CH_3—$ | 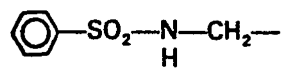 |
| $CH_3—$ | $HO—CH_2—$ |
| $CH_3—$ | $H_5C_2—COO—CH_2—$ |
| $CH_3—$ | $—OH$ |
| $CH_3—$ | $—SO_3H$ |
| $CH_3—$ | $—CN$ |
| $CH_3—$ | $—OCH_3$ |
| $CH_3—$ | $—O—CH_2—CH_2—CH_2—CH_3$ |
| $CH_3—$ | $—SH$ |
| $CH_3—$ | $—S—CH_2—CH_3$ |
| $CH_3—$ | $—NH_2$ |
| $CH_3—$ | $—NH—CH_3$ |

Verbindungen der Formel

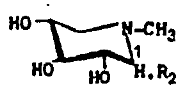

Die nachstehend aufgeführten Beispiele umfassen bezüglich der Konfiguration am C-1-Atom sowohl $\alpha$- als auch $\beta$-Form.

| $R_2$ |
|---|
| $H_2N—CH_2—$ |
| $CH_3CO—NH—CH_2—$ |
| 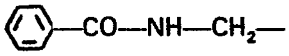 |
| 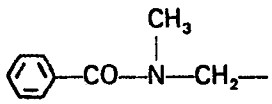 |
| $CH_3NHCONH—CH_2—$ |
| 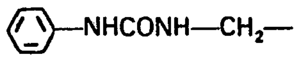 |
| 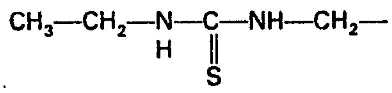 |
| $C_2H_5OCONH—CH_2—$ |
| $—COOH$ |
| $—COOC_2H_5$ |
| $—CONH_2$ |
| 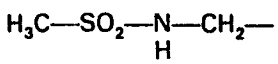 |
| 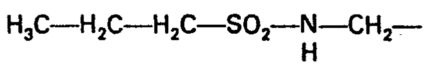 |
| 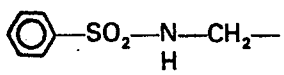 |
| $HO—CH_2—$ |
| 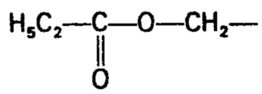 |

| $R_2$ |
|---|
| $—OH$ |
| $—CN$ |
| $—SO_3H$ |
| $—OCH_3$ |

| $R_2$ |
|---|
| $—O—CH_2—CH_2—CH_2—CH_3$ |
| $—SH$ |
| $—S—CH_2—CH_3$ |
| $—NH_2$ |
| $—NH—CH_3$ |

Verbindungen der Formel

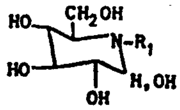

| $R_1$ |
| --- |
| $—CH_2—CH_3$ |
| $—CH_2—CH_2—CH_2—CH_3—$ |
| $—CH_2—(CH_2)_{16}—CH_3—$ |
| 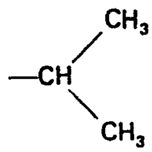 |
| 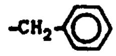 |
| $—CH_2—CH{=}CH_2—$ |
| $—CH_2—CH_2—OCH_3—$ |
| 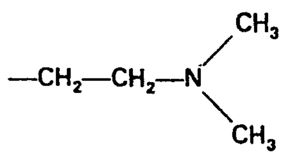 |
| 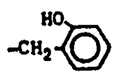 |

Verbindungen der Formel

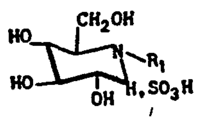

| $R_1$ |
| --- |
| $—CH_2—CH_3$ |
| $—CH_2—CH_2—CH_2—CH_3$ |
| $—CH_2—(CH_2)_{16}—CH_3$ |
| 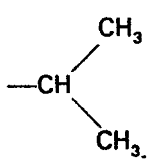 |
| 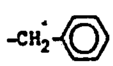 |
| $—CH_2—CH{=}CH_2$ |
| $—CH_2—CH_2—OCH_3$ |
| 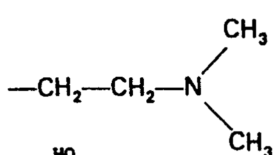 |
| 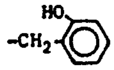 |

Verbindungen der Formel

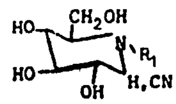

$R_1$

$-CH_2-CH_3$

$-CH_2-CH_2-CH_2-CH_3$

$-CH_2-(CH_2)_{16}-CH_3$

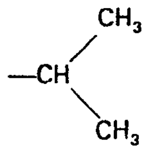

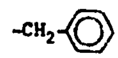

$-CH_2-CH=CH_2$

$-CH_2-CH_2-OCH_3$

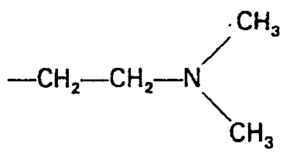

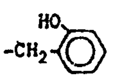

Die erfindungsgemäßen Inhibitoren eignen sich als Therapeutica für folgende Indikationen: Prädiabetes, Gastritis, Obstipation, Karies, Infektionen des Gastro-Intestinaltraktes, Meteorismus, Flatulenz, Hypertension, Atheroskelerose und besonders Adipositas, Diabetes und Hyperlipoprotämie.

Zur Verbreiterung des Wirkungsspektrums kann es sich empfehlen, Inhibitoren für Glycosidhydrolasen, die sich gegenseitig in ihrer Wirkung ergänzen, zu kombinieren, sei es, daß es sich um Kombinationen der erfindungsgemäßen Inhibitoren untereinander oder um Kombinationan der erfindungsgemäßen Inhibitoren mit bereits bekannten handelt. So kann es beispielsweise zweckmäßig sein erfindungsgemäße Saccharase-Inhibitoren mit bereits bekannten Amylase-Inhibitoren zu kombinieren.

Vorteilhaft sind in manchen Fällen auch Kombinationen der erfindungsgemäßen Inhibitoren mit bekannten oralen Antidiabetica ($\beta$-cytotrope Sulfonylharnstoffderivate und/oder blutzuckerwirksame Biguanide) sowie mit blutlipid-senkenden Wirkstoffen wie z.B. Clofibrat, Nicotinsäure, Cholestyramin und andere.

Die Verbindungen können ohne Verdünnung, z.B. als Pulver oder in einer Gelatinehülle oder in Kombination mit einem Trägerstoff in einer pharmazeutischen Zusammensetzung appliziert werden.

Pharmazeutische Zubereitungen können eine größere oder kleinere Menge des Inhibitors enthalten, z.B. 0,1% bis 99,5%, in Kombination mit einem pharmazeutisch verträglichen nichttoxischen, inerten Trägerstoff, wobei der Trägerstoff eine oder mehrere feste, halbfeste oder flüssige Verdünnungsmittel, Füllstoffe und/oder nichttoxisches, inertes und pharmazeutisch-verträgliches Formulierungshilfsmittel enthalten kann. Solche pharmazeutischen Zubereitungen liegen vorzugsweise in Form von Dosierungseinheiten vor, d. h. physikalisch-diskreten, eine bestimmte Menge des Inhibitors enthaltenden Einheiten, die einem Bruchteil oder einem Vielfachen der Dosis entsprechen, die zur Herbeiführung der gewünschten Hemmwirkung erforderlich sind. Die Dosierungseinheiten können 1, 2, 3, 4 oder mehr Einzeldosen oder 1/2, 1/3 oder 1/4 einer Einzeldosis enthalten. Eine Einzeldosis enthält vorzugsweise eine genügende Menge Wirkstoff, um bei einer Applikation gemäß eines vorher bestimmten Dosierungsschemas einer oder mehrerer Dosierungseinheiten die gewünschte Hemmwirkung zu erzielen, wobei eine ganze, eine halbe, oder ein Drittel oder ein Viertel der Tagesdosis gewöhnlich zu allen, Haupt- und Nebenmahlzeiten am Tage verabreicht wird. Andere therapeutische Mittel können auch eingenommen werden. Obgleich die Dosierung und das Dosierungsschema in jedem Fall sorgsam abgewogen werden sollte, unter Anwendung gründlichen fachmännischen Urteils und unter Beachtung des Alters, des Gewichts und des Zustands des Patienten, der Art und der Schwere der Erkrankung, wird die Dosierung gewöhnlich in einem Bereich zwischen etwa 1 bis etwa $1 \times 10^4$ SIE/kg des Körpergewichtes pro Tag liegen. In manchen Fällen wird man dabei eine ausreichende therapeutische

Wirkung mit einer geringeren Dosis erreichen, während in anderen Fällen eine größere Dosis erforderlich sein wird.

Orale Applikation kann unter Verwendung fester und flüssiger Dosierungseinheiten durchgeführt werden, wie z.B. Pulver, Tabletten, Dragees, Kapseln, Granulate, Suspensionen, Lösungen und dergleichen.

Pulver wird durch Zerkleinerung der Substanz in einer geigneten Größe und Vermischen mit einem ebenfalls zerkleinerten pharmazeutischen Trägerstoff hergestellt. Obgleich ein eßbares Kohlenhydrat, wie z. B. Stärke, Lactose, Saccharose oder Glucose normalerweise zu diesem Zwecke Verwendung findet und auch hier benutzt werden kann, ist es wünschenswert ein nicht metabolisierbares Kohlenhydrat, wie z. B. ein Cellulosederivat zu benutzen.

Süßmittel, Geschmackszusätze, Konservierungsstoffe, Dispergiermittel und Färbemittel können auch mitverwendet werden.

Die Kapseln können durch Zubereitung der oben beschriebenen Pulvermischung und durch Füllung bereits gebildeter Gelatinehüllen hergestellt werden. Die Pulvermischung kann man vor dem Füllvorgang mit Gleitmitteln, wie z. B. Kieselgel, Talkum, Magnesiumstearat, Calciumstearat oder festem Polyäthylenglykol versetzen. Die Mischung kann man ebenfalls mit einem Desintegrator oder Lösungsvermittler, wie z. B. Agar-Agar, Calciumcarbonat oder Natriumcarbonat versetzen, um bei Einnahme der Kapsel die Zugänglichkeit des Inhibitors zu verbessern.

Die Anfertigung der Tabletten erfolgt zum Beispiel durch Herstellung einer Pulvermischung, grob oder feinkörnig, und Hinzfügung eines Gleitmittels und Desintegrators. Aus dieser Mischung formt man Tabletten. Eine Pulvermischung bereitet man vor durch Mischung der Substanz, welche in geeigneter Weise zerkleinert wurde und ergänzt ein Verdünnungsmittel oder eine andere Trägersubstanz wie oben beschreiben. Gegebenenfalls fügt man ein Bindemittel hinzu: z.B. Carboxymethylcellulose, Alginate, Gelatine oder Polyvinylpyrrolidone, einen Lösungsverzögerer, wie z. B. Paraffin, einen Resorptionsbeschleuniger, wie z. B. ein quarternäres Salz und/oder ein Adsorptionsmittel, wie z. B. Bentonit, Kaolin oder Dicalciumphosphat. Die Pulvermischung kann granuliert werden zusammen mit einem Bindemittel, wie z. B. Sirup, Stärkepaste, Akazienschleim, oder Lösungen aus Zellulose- oder Polymerenmaterialien. Danach preßt man das Produkt durch ein grobes Sieb. Als Alternative hierzu kann man die Pulvermischung durch eine Tablettenmaschine laufen lassen und die sich ergebenden ungleichmäßig geformten Stücke bis auf Korngröße zerkleinern. Damit die entstandenen Körner nicht in den tablettenbildenden Düsen stecken bleiben, kann man sie mit einem Gleitmittel versetzen, wie z. B. Stearinsäure, Stearatsalz, Talkum oder Mineralöl. Diese gleitfähig gemachte Mischung wird dann in Tablettenform gepreßt. Die Wirkstoffe können auch mit freifließenden inerten Trägerstoffen vereinigt werden und direkt in Tablettenform gebracht werden unter Auslassung der Granulat-oder Zerstuckelungsschritte. Man kann das Produkt mit einer klaren oder opaken Schutzhülle versehen, z. B. einem Überzug aus Schellack, einem Überzug aus Zucker oder Polymersubstanzen und einer polierten Hülle aus Wachs. Farbstoffe können diesen Überzügen beigefügt werden, damit zwischen den verschiedenen Dosierungseinheiten unterschieden werden kann.

Die oral zu verabreichenden Zubereitungsformen, wie z. B. Lösungen, Syrup und Elixire, lassen sich in Dosierungseinheiten herstellen, so daß eine bestimmte Menge Präparat eine bestimmte Menge Wirkstoff enthält. Syrup kann so hergestellt werden, daß der Wirkstoff in einer wäßrigen Lösung, welche geeignete Geschmackstoffe enthält, gelöst wird; Elixire werden unter Verwendung nichttoxischer, alkoholischer Trägerstoffe erhalten. Suspensionen kann man durch Dispergieren der Verbindung in einem nicht toxischen Trägerstoff darstellen. Lösungsvermittler und Emulgiermittel, wie z. B. äthoxylierte Isostearylalkohole und Polyoxyäthylensorbitester, Konservierungsmittel, geschmacksverbessernde Zusätze wie z. B. Pfefferminzöl oder Saccharin und dergl. können auch zugegeben werden.

Dosierungsvorschriften können auf der Kapsel angegeben werden. Überdies kann die Dosierung so abgesichert sein, daß der Wirkstoff verzögert abgegeben wird, z. B. durch Einhalten des Wirkstoffes in Polymerensubstanzen, Wachse oder dergl.

Zusätzlich zu den oben erwähnten pharmazeutischen Zusammensetzungen lassen sich auch diese Wirkstoffe enthaltende Lebensmittel herstellen; beispielsweise Zucker, Brot, Kartoffelprodukte, Fuchtsaft, Bier, Schokolade und andere Konfektartikel, und Konserven, wie z. B. Marmelade, wobei zu diesen Produkten eine therapeutisch-wirksame Menge mindestens eines der erfindungsgemäßen Inhibitoren gegeben wurde.

Die unter Verwendung der erfindungsgemäßen Wirkstoffe hergestellten Nahrungsmittel eignen sich sowohl zur Diät bei Patienten, die an Stoffwechselstörungen leiden als auch zur Ernährung gesunder Personen im Sinne einer Stoffwechselstörungen vorbeugenden Ernährungsweise.

Die erfindungsgemäßen Inhibitoren weisen weiterhin die Eigenschaft auf, in Tieren das Verhältnis des Anteiles an unerwünschtem Fett zum Anteil des erwünschten fettarmen Fleisches (mageres Fleisch) zugunsten des mageren Fleisches in hohem Maße zu beeinflussen. Dies ist von besonderer Bedeutung für die Aufzucht und Haltung von landwirtschaftlichen Nutztieren, z. B. in der Schweinemast, aber auch von erheblicher Bedeutung für die Aufzucht und Haltung von sonstigen Nutztieren und Ziertieren. Die Verwundung der Inhibitoren kann weiterhin zu einer erheblichen Rationalisierung der Fütterung der Tiere führen, sowohl zeitlich, mengenmäßig wie auch qualitätsmäßig. Da sie eine gewisse Verzögerung der

Verdauung bewirken, wird die Verweildauer der Nährstoffe im Verdauungstrakt verlängert, wodurch eine mit weniger Aufwand verbundene ad libitum-Fütterung ermöglicht wird. Weiterhin ergibt sich bei der Verwendung der erfindungsgemäßen Inhibitoren in vielen Fällen eine erhebliche Einsparung von wertvollem Proteinfutter.

Die Wirkstoffe können somit praktisch in allen Bereichen der Tierernährung als Mittel zur Reduzierung des Fettansatzes sowie der Einsparung von Futtereiweiß verwendet werden.

Die Wirksamkeit der Wirkstoffe ist hierbei weitgehend unabhängig von der Art und dem Geschlecht der Tiere. Besonders wertvoll erweisen sich die Wirkstoffe bein Tierarten, die überhaupt oder in bestimmten Lebensabschnitten zu stärkerer Fetteinlagerung neigen.

Als Tiere, bei denen die Inhibitoren zur Reduzierung des Fettansatzes und/oder zur Einsparung von Futtereiweiß eingesetzt werden können, seien beispielsweise folgende Nutz- und Ziertiere genannt: Warmblüter wie Rinder, Schweine, Pferde, Schafe, Ziegen, Katzen, Hunde, Kaninchen, Pelztiere, z. B. Nerze, Chinchilla, andere Ziertiere, z. B. Meerschweinchen und Hamster, Labor- und Zootiere, z. B. Ratten, Mäuse, Affen usw. Geflügel, z. B. Broiler, Hühner, Gänse, Enten, Truthähne, Tauben, Papageien und Kanarienvögel und Kaltblüter, wie Fische, z. B. Karpfen und Reptilien, z. B. Schlangen.

Die Menge der Wirkstoffe, die den Tieren zur Erriechung des gewünschten Effektes verabreicht wird, kann wegen der günstigen Eigenschaften der Wirkstoffe weitgehend variiert werden. Sie liegt vorzugsweise bei etwa 0,5 mg bis 2,5 g, insbesondere 10 bis 100 mg/kg Futter pro Tag. Die Dauer der Verabriechung kann von wenigen Stunden oder Taben bis zu mehreren Jahren betragen. Die passende Menge Wirkstoff sowie die passende Dauer der Verabreichung stehen in engem Zusammenhang mit dem Fütterungsziel. Sie hängen insbesondere von der Art, dem Alter, dem Geschlecht, dem Gesundheitszustand und der Art der Haltung der Tiere ab und sind durch jeden Fachmann leicht zu ermitteln.

Die erfindungsgemäßen Wirkstoffe werden den Tieren nach den üblichen Methoden verabreicht. Die Art der Verabreichung hängt insbesondere von der Art., dem Verhalten und dem Allgemeinzustand der Tiere ab. So kann die Verabreichung einmal oder mehrmals täglich, in regelmäßigen oder unregelmäßigen Abständen, oral erfolgen. Aus Zwickmäßrigkeitsgründen ist in den meisten Fällen eine orale Verabreichung, insbesondere im Rhythmus der Nahrungs- und/oder Getränkeaufnahme der Tiere, vorzuziehen.

Die Wirkstoffe können als reine Stoffe oder in formulierter Form verabreicht werden, wobei die formulierte Form sowohl als Premix, also in Mischung mit nichttoxischen inerten Trägerstoffen beliebiger Art, als auch als Teil einer Gesamtration in Form eines Beifutters bzw. als Mischungsbestandteil eines alleinigen Mischfutters zu verstehen ist. Mit eingeschlossen ist auch die Applikation geeigneter Zubereitungen über das Trinkwasser.

Die Wirkstoffe können gegebenenfalls in formulierter Form auch zusammen mit anderen Nähr- und Wirkstoffen, z. B. Mineralsalzen, Spurenelementen, Vitaminen, Eiweißstoffen, Energieträgern (z. B. Stärke, Zucker, Fette), Farbstoffen und/oder Geschmacksstoffen oder anderen Futterzusatzstoffen, wie z. B. Wachstumsförderern, in geeigneter Form verabreicht werden. Die Wirstoffe können der Tieren vor, während oder nach der Nahrungsaufnahme gegeben werden.

Empfehlenswert ist die orale Verabreichung zusammen mit dem Futter und/oder Trinkwasser, wobei je nach Bedarf die Wirkstoffe der Gesamtmenge oder nur Teilen des Futters und/oder Trinkwassers zugegeben werden.

Die Wirkstoffe können nach üblichen Methoden durch einfaches Mischen als reine Stoffe, vorzugsweise in fein verteilter Form oder in formulierter Form in Mischung mit eßbaren, nichttoxischen Trägerstoffen, gegebenenfalls auch in Form eines Premix oder eines Futterkonzentrates, dem Futter und/oder dem Trinkwasser beigefügt werden.

Das Futter und/oder Trinkwasser kann beispielsweise die erfindungsgemäßen Wirkstoffe in einer Konzentration von etwa 0,001 bis 5,0%, insbesondere 0,02 bis 2,0% (Gewicht) enthalten. Die optimale Höhe der Konzentration des Wirkstoffs im Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen, handelsüblichen oder speziellen Futterzusammensetzungen verwendet werden, die vorzugsweise das übliche, für eine ausgewogene Ernährung notwendige Gleichgewicht aus Energie- und Eiweißstoffen, einschließlich Vitaminen und Mineralstoffen enthalten. Das Futter kann sich beispielsweise zusammensetzen aus pflanzlichen Stoffen, z. B. Ölkuckenschroten, Getreideschroten, Getreidenebenprodukten, aber auch aus Heu, Gärfutter, Rüben und anderen Futterpflanzen, aus tierischen Stoffe, z. B. Fleisch- und Fisch-produkte, Knochenmehl, Fette, Vitamine, z. B. A, D, E, K und B-Komplex sowie spezielle Proteinquellen, z. B. Hefen sowie bestimmte Aminosäuren und Mineralstoffen und Spurenelementen, wie z. B. Phosphor und Eisen, Zink, Mangan, Kupfer, Kobalt, Jod usw.

Premixe können vorzugsweise etwa 0,1 bis 50%, insbesondere 0,5 bis 5,0% (Gewicht) z.B. N-Methyl-1-desoxynojirimycin neben beliebigen eßbaren Trägerstoffen und/oder Mineralsalzen, z.B. kohlensaurem Futterkalk enthalten und werden nach den üblichen Mischmethoden hergestellt.

Mischfutter enthalten vorzugsweise 0,001 bis 5,0%, insbesondere 0,02 bis 2,0% (Gewicht) beispielsweise an N-Methyl-1-desoxynojirimycin neben den üblichen Rohstoffkomponenten eines Mischfutters, z. B. Getreideschrote oder -nebenprodukte, Ölkuchenschrote, tierisches Eiweiß, Mineralien, Spurenelemente und Vitamine. Sie können nach den üblichen Mischmethoden hergestellt werden.

Vorzugsweise in Premixen und Mischfuttermitteln können die Wirkstoffe gegebenenfalls auch durch ihre Oberfläche bedeckenden geeigneten Mittel, z. B. mit nichttoxischen Wachsen oder Gelatine vor Luft, Licht und/oder Feuchtigkeit geschützt werden.

Beispiel für die Zusammensetzung eines fertigen Mischfutters, für Geflügel, das einen erfindungsgemäßen Wirkstoff enthält:

200 g Weizen, 340 g Mais, 360,3 g Sojaschrot, 60 g Rindertalg, 15 g Dicalciumphosphat, 10 g Calciumcarbonat, 4 g jodiertes Kochsalz, 7,5 g Vitamin-Mineral-Mischung und 3,2 g Wirkstoff-Premix ergeben nach sorgfältigem Mischen 1 kg Futter.

Die Vitamin-Mineral-Mischung besteht aus:

6000 I.E. Vitamin A, 1000 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4 \times H_2O$, 140 mg Zn $SO_4 \times 7H_2O$, 100 mg Fe $SO_4 \times 7H_2O$ und 20 mg Cu $SO_4 \times 5H_2O$. Der Wirkstoff-Premix enthält z. B. N-Methyl-1-desoxynojirimycin in der gewünschten Menge, z. B. 1600 mg und zusätzlich 1 g DL-Methionin sowie so viel Sojabohnenmehl, daß 3,2 g Premix entstehen.

Beispiel für die Zusammensetzung eines Schweinemischfutters, das einen Wirkstoff der Formel I enthält:

630 g Futtergetreideschrot (zusammengesetzt aus 200 g Mais-, 150 g Gerste-, 150 g Hafer- und 130 g Weizenschrot), 80 g Fischmehl, 60 g Sojaschrot, 58,8 g Tapiokamehl, 8 g Bierhefe, 50 g Vitamin-Mineral-Mischung für Schweine (Zusammensetzung, z. B. wie beim Kükenfutter) 30 g Leinkuchenmehl, 30 g Maiskelberfutter, 10 g Sojaöl, 10 g Zuckerrohrmelasse und 2 g Wirkstoff-Premix (Zusammensetzung z. B. beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

Die Inhibitoren können einzeln oder aber auch in beliebigen Mischungen untereinander verwendet werden.

*Saccharase-Inhibitionstest in vitro*

Der Saccharase-Inhibitionstest in vitro ermöglicht die Bestimmung der enzyminhibitorischen Aktivität einer Substanz durch den Vergleich der Aktivität des solubilisierten intestinalen Disaccharidasen-Komplexes in Gegenwart bzw. in Abwesenheit (sog. 100%-Wert) des Inhibitors. Als Substrat, welches die Spezifität des Inhibitionstestes bestimmt, dient dabei eine praktisch Glucose-freie Saccharose (Glucose <100 ppm); die Enzymaktivitätsbestimmung basiert auf der spektrophotometrischen Bestimmung freigesetzter Glucose mittels Glucose-Dehydrogenase und Nicotinamid-adenin-dinucleotid als Cofaktor.

Eine Saccharase-Inhibitor-Einheit (SIE) ist definiert als diejenige inhibitorische Aktivität, welche in einem definierten Testansatz eine vorgegebene saccharolytische Aktivität um eine Einheit (Saccharase-Einheit = SE) reduziert; die Saccharase-Einheit ist dabei als diejenige Enzymaktivität definiert, welche unter vorgegebenen Bedingungen ein $\mu$mol Saccharose pro min spaltet und damit zur Freisetzung von je ein $\mu$mol Glucose, welche im Test bestimmt wird, und Fructose, welche im Test nicht erfaßt wird, führt.

Der intestinale Disaccharidasen-Komplex wird aus Schweine-dünndarm-Mucosa durch tryptische Verdauung, Fällung aus 66% Äthanol bei −20°C, Aufnehmen des Präcipitates in 100 mM Phosphat-Puffer, pH 7,0 und abschließende Dialyse gegen denselben Puffer gewonnen.

10 $\mu$l einer Probelösung, die so angesetzt ist, daß die Extinktion des Testansatzes mindestens 10%, jedoch nicht mehr als 25% unter der des 100%-Wertes liegt, werden mit 100 $\mu$l einer Verdünnung des intestinalen Disaccharidasen-Komplexes in 0,1 M Maleinat-Puffer, pH 6,25, versetzt and für 10 min bei 37°C vorinkubiert. Die Verdünnung des Disaccharidasen-Komplexes ist auf eine Aktivität von 0,1 SE/ml einzustellen.

Anschließend wird die saccharolytische Reaktion durch Zugabe von 100 $\mu$l einer 0,4 M Lösung von Saccharose ("SERVA 35579") in 0,1 M Maleinat-Puffer, pH 6,25 gestartet und nach einer Inkubationsdauer von 20 min bei 37°C durch die Zugabe von 1 ml Glucose-Dehydrogenase-Reagenz (1 Fläschchen Glucose-Dehydrogenase-Mutarotase-Gemisch lyophiliert ("MERCK 14035") und 331,7 mg $\beta$-Nicotinamid-adenin-dinucleotid (freie Säure, "BOEHRINGER" Reinheitsgrad I) in 250 ml 0,5 M Tris-Puffer, pH 7,6 gelöst) abgestoppt. Zum Nachweis der Glucose wird 30 min bei 37°C inkubiert und schließlich bei 340 nm gegen einem Reagenzienblank (mit Enzym, jedoch ohne Saccharose) photometriert.

Die Berechnung der Hemmaktivität von Inhibitoren ist dadurch erschwert, daß schon geringfügige Änderungen im Testsystem, beispielsweise ein geringfügig von Bestimmung zu Bestimmung variierender 100%-Wert, von nicht mehr zu vernachlässigendem Einfluß auf das Testergebnis sind. Man umgeht diese Schwierigkeiten, indem man bei jeder Bestimmung einen Standard mitlaufen läßt; als Standard dient ein Saccharase-Inhibitor der Formel $C_{25}H_{43}O_{18}N$, welcher eine spezifische Hemmaktivi-

tät von 77 700 SIE/g aufweist und bei eingesetzten Mengen von 10 bis 20 ng im Test zu einer Hemmung von oben spiezifizierter Größenordnung führt. Bei Kenntnis der Differenz der Extinktionen bei 340 nm von 100%-Wert und durch Standard gehemmtem Ansatz läßt sich aus der Extinktions-differenz von 100%-Wert und durch die Probelösung gehemmtem Ansatz unter Berücksichtigung der eingesetzten Menge an Inhibitor in bekannter Weise dessen spezifische Hemmaktivität errechnen, ausgedrückt in Saccharase-Inhibitor-Einheiten pro Gramm (SIE/g).

*Spezifische saccharaseinhibitorische Aktivität in vitro*

| | |
|---|---|
| 1-Desoxynojirimycin | 465 000 SIE/g |
| N-Methyl-1-desoxynojirimycin (hier nicht beansprucht) | 2 330 000 SIE/g |

Herstellungsbeispiele

Beispiel 1

*N-Methyl-1-desoxynojirimycin* (hier nicht beansprucht)

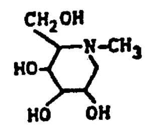

Zu 4 ml 98%iger Ameisensäure gibt man unter Eiskühlung 3,2 g 1-Desoxynojirimycin und 2 ml 30%igen wäßrigen Formaldehyd. Anschließend wird 8 Stunden under Rückfluß erhitzt. Nach dem Abkühlen verdünnt man das Reaktionsgemisch mit Aceton. Ex fällt ein harzartiger Niederschlag aus. Man dekantiert die Acetonlösung ab und wäscht das Harz mehrfach mit Aceton nach. Der Rückstand wird anschließend in destilliertem Wasser gelöst und die Lösung durch Zugabe von basischem Ionenaustauscher in der ⊖OH-Form (Amberlite JRA 410) von Ameisensäure befreit. Der Ionenaustauscher wird abfiltriert und die wäßrige Lösung unter vermindertem Druck zur Trockne gebracht. Zurück bleiben 3,0 g harziges N-Methyl-1-Desoxynojirimycin. Die Verbindung kann durch Chromatographie an Cellulose weiter gereinigt werden. Als Fließmittel wird wasserhaltiges Butanol verwendet.
Schmelzpunkt: 153°C (Äthanol).

Beispiel 2

*N-n-Butyl-1-desoxynojirimycin*

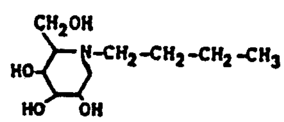

Zu 3,2 g 1-Desoxynojirimycin (0,02 Mol) in 40 ml absolutem Methanol gibt man nacheinander unter Eiskühlung und Rühren 12,5 ml n-Butyraldehyd 0,01 Mol methanolische HCl und 1,5 g $NaCNBH_3$. Das Reaktionsgemisch wird 12 Stunden bei Raumtemperatur gerührt. Dann engt man am Rotationsverdampfer zur Trockne ein. Der Rückstand wird in 50 ml Wasser gelöst und 3 x mit je 30 ml $CHCl_3$, extrahiert. Die wäßrige Phase wird erneut zur Trocckne gebracht, der Rückstand wird in 30 ml $H_2O$ aufgenommen und auf eine 50 cm lange und 2 cm weite Säule aufgetragen, die mit stark basischem Ionenaustauscher in der OH⊖-Form (Amberlite IRA 400 oder Dowex 1 x 2) gefüllt ist.

Es wird mit Wasser eluiert und die einzelnen Fraktionen werden dünnschichtchromatographisch untersucht. (Kieselgelplatten; Laufmittel:Essigester/Methanol/Wasser/25%iger Ammoniak 100:60:40:2; Sprühreagenz: $KMnO_4$-Lösung). Die Fraktionen, die N-n-Butyl-1-desoxynojirimycin enthalten werden zusammengefaßt und die wäßrige Lösung am Rotationsverdampfer eingeengt. Der Rückstand wird mit Aceton versetzt, wobei Kristallisation eintritt.

Die Kristalle werden abfesaugt, mit Aceton kurz nachgewaschen und getrocknet. Es werden 3 g N-n-Butyl-1-desoxynojirimycin vom Schmelzpunkt 126—127°C erhalten.

*Massenspektrum:* Die wichtigsten Peaks im oberen Massenbereich findet man bei m/e = 188 ($M—CH_2OH$) und m/e = 176 ($M—CH_2—CH_2—CH_3$).

Bei weniger reaktionsfähigen Aldehyden wurde dem Reaktionsansatz zur Bindung des Reaktionswassers Molekularsieb 3Å zugesetzt.

Analog zu dieser Vorschrift wurden hergestellt:

*N-n-Heptyl-1-desoxynojirimycin*

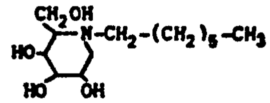

Schmelzpunkt: 111—113°C (Aceton).
*Massenspektrum:* Der wichtigste Peak im oberen Massenbereich liegt bei m/e = 230 ($M—CH_2OH$). Außerdem findet man Peaks bei m/e = 262 (M+H) und 260 (M—H).

*N-Benzyl-1-desoxynojirimycin*

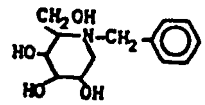

*Massenspektrum:* Den wichtigsten Peak im oberen Massenbereich findet man bei m/e = 222 ($M—CH_2OH$).
Schmelzpunkt: 183—184°C (Methanol).

*N-(2-Pyridyl)-methyl-1-desoxynojirimycin*

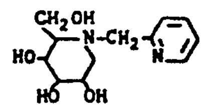

*Massenspektrum:* Die wichtigsten Peaks im oberen Massenbereich findet man bei m/e = 255 (M+H), m/e = 236 ($M—H_2O$) und m/e = 223 ($M—CH_2OH$).
Schmelzpunkt: 174—175°C (Athanol)

*N-2-Hydroxyäthyl-1-desoxyjojirimycin*

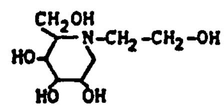

Schmelzpunkt: 114°C (Äthanol)
*Massenspektrum:* Der wichtigste Peak im oberen Massenbereich liegt bei m/e = 176 ($M—CH_2OH$).

*N-2,3-Dihydroxy-n-propyl-1-desoxynojirimycin*

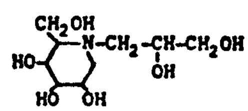

*Massenspektrum:* Die wichtigsten Peaks im oberen Massenbereich liegen bei m/e = 206 ($M—CH_2OH$) und m/e = 176. Die Substanz ist ein Gemisch zweier diastereomerer Verbindungen.

*N-(S-β-D-Glucopyranosyl-2-mercaptoäthyl)-1-desoxynojirimycin*

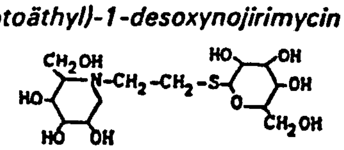

*Massenspektrum:* Das Massenspektrum wurde von der in Pyridin/Acetanhydrid peracetylierten Verbindung gemessen.
Die wichtigsten Peaks im oberen Massenbereich findet man bei m/e = 648

$$(M—CH_2O—\underset{\underset{O}{\|}}{C}—CH_3),$$

m/e = 588 und m/e = 344.

Der für die Umsetzung benötigte Aldehyd wurde aus O-acetylierter 1-Thioglucose und Chloracetaldehyd gewonnen. Die Abspaltung der Acetylgruppen erfolgte im Endprodukt durch Umesterung mit katalytischen Mengen $NaOCH_3$ in MeOH.

*N-Oxiranyl-methyl-1-desoxynojirimycin*

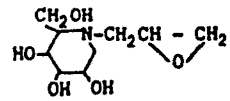

*Massenspektrum:* Die wichtigsten Peaks in oberen Massenbereich findet man bei m/e = 219 (M), m/e = 202, m/e = 188 (M—$CH_2OH$) und m/e = 176

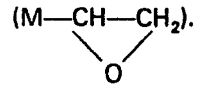

Die Substanz ist ein Gemisch zweier diastereomerer Verbindungen.

*N-(3-N-Phthalimido-n-propyl)-1-desoxynojirimycin*

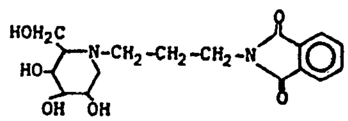

*Massenspektrum:* Die wichtigsten Peaks im oberen Massenbereich wurden bei m/e = 348, m/e = 319 (M—$CH_2OH$), m/e = 301, m/e = 200, m/e = 188, m/e = 174, m/e 160 und m/e = 147 gefunden.

In diesem Fall wurde auf die Chromatographie an basischen Ionenaustauscher verzichtet und die Verbindung durch Auskochen mit Aceton und Umkristallisation aus Aethanol gereinigt.
F.P.: 208—210°C.

*N-(3-Amino-n-propyl)-1-desoxynojirimycin*

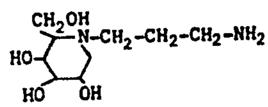

*Massenspektrum:* Die wichtigsten Peaks im oberen Massenbereich liegen bei m/e = 189 (M—$CH_2OH$) und m/e = 146.

Die Verbindung wurde aus obiger Phthalimidoverbindung durch Hydrazinolyse in Methanol gewonnen.

*N-(1-Desoxynojirimycin-yl)-essigsäure*

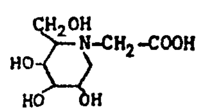

*Massenspektrum:* Die wichtigsten Peaks im oberen Massenbereich findet man bei m/e = 203 (M—$H_2O$), m/e = 159, m/e = 145 und m/e = 100.

Die Reinigung der Verbindung erfolgte nicht durch Chromatographie über basischen Austauscher, sondern durch Umkristallisation aus Methanol/Wasser.
F.P.: 187—188°C.

*N-o-Nitrobenzyl-1-desoxynojirimycin*

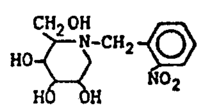

Rf-Wert: 0,85 (auf DC-Fertigplatten der Firma Merck Kieselgel 60; Fließmittel:Essigester/Methanol/$H_2O$/25%iger Ammoniak 100:60:40:2).
Zum Vergleich: Rf-Wert von 1-Desoxynojirimycin: 0,3.

*N-o-Carboxybenzyl-1-desoxynojirimycin*

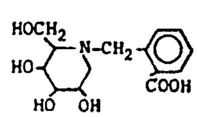

Rf-Wert: 0,7 (Platten und Fließmittel wie bei vorstehender Verbindung angegeben).

Zur Reinigung wurde die Verbindung wie oben angegeben über basischen chromatographiert, wobei aber zum Schluß mit 1%iger Essigsäure eluiert wurde.

*N-p-Carboxybenzyl-1-desoxynojirimycin*

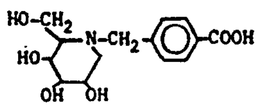

Rf-Wert: 0,7 (Platten und Fließmittel wie oben angegeben). Auch hier wurde die Verbindung mit 1%iger Essigsäure vom basischen Austauscher eluiert.
Schmelzpunkt: 280—281°C (Methanol)

*N-p-sulfobenzyl-1-desoxynojirimycin*

Zu 2 g 1-Desoxynojirimycin in 40 ml Methanol wurden 4,8 g Benzaldehyd-4-sulfonsäure, 1,8 ml Eisessig und 0.8 g, $NaCNBH_3$ gegeben. Es wurde 4 Stunden unter Rückfluß erhitzt und über Nacht bei Raumtemperaturen gerührt. Das ausgefallene Reaktionsprodukt wurde abgesaugt und aus Wasser umkristallisiert. Ausbeute: 1,2 g Schmelzpunkt: ~320°C (Zersetzung).

Beispiel 3

N-β-Phenylethyl-1-desoxynojirimycin

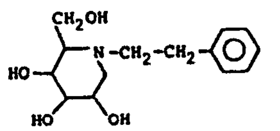

Zu 2 g 1-Desoxynojirimycin und 1,8 ml Essigsäure in 40 ml Methanol gab man 3 g Phenylacetaldehyd und 0,8 g $NaCNBH_3$. Anschließend wurde über Nacht bei Raumtemperatur gerührt. Das Reaktionsgemisch wurde am Rotationsverdampfer zur Trockene gebracht. Der Rückstand wurde in Ethanol/$H_2O$ 2:1 gelöst und auf eine mit stark saurem Ionenaustauscher in der $H^{\ominus}$-Form (Amberlite IR 120) gefüllte Säule aufgetragen. Die Säule wurde mit 2 L Ethanol/$H_2O$ 2:1 gewaschen. Anschließend wurde das Reaktionsprodukt mit Ethanol/2%-igem wäßrigem $NH_3$ 2:1 von der Säule eluiert. Die einzelnen Fraktionen wurden dünnschichtchromatographisch untersucht und diejenigen, die N-β-Phenylethyl-1-desoxynojirimycin enthielten, zusammengefaßt und zur Trockene gebracht. Der Rückstand wurde aus ca. 100 ml Ethanol kristallisiert. Ausbeute: 2,5 g N-β-Phenylethyl-1-desoxynojirimycin vom Schmelzpunkt 179—181°C.

Auf analogem Wege wurden hergestellt:

N-n-Pentyl-1-desoxynojirimycin

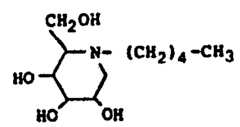

F.P.: 97°C (aus Aceton)

N-n-Hexyl-1-desoxynojirimycin

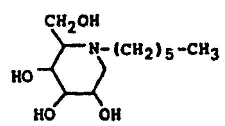

F.P.: 112—113°C (aus Ethanol/Aceton)

N-n-Octyl-1-desoxynojirimycin

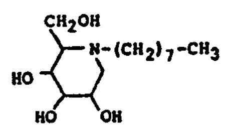

F.P.: 115—117°C (aus Ethanol/Aceton)

N-n-Nonyl-1-desoxynojirimycin

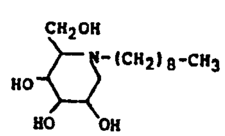

F.P.: 105—107°C (aus Ethanol/Aceton)

N-n-Decyl-1-desoxynojirimycin

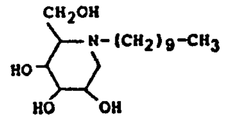

F.P.: 151°C (sintert bei 91°C, aus MeOH/Aceton)

N-n-Undecyl-1-desoxynojirimycin

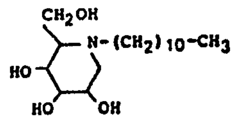

F.P.: 162°C (sintert bei 97°C, aus Ethanol/Aceton)

N-n-Dodecyl-1-desoxynojirimycin

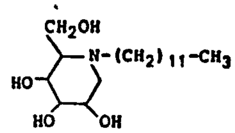

F.P.: 164°C (sintert bei 97°C, aus Ethanol/Aceton)

N-n-Tetradecyl-1-desoxynojirimycin

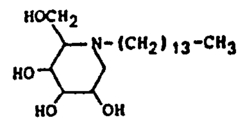

F.P.: 105—107°C (aus Methanol)

N-n-(5'-Hydroxypentyl)-1-desoxynojirimycin

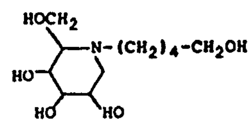

F.P.: 86—87°C (aus Butanol)

N-Cyclohexylmethyl-1-desoxynojirimycin

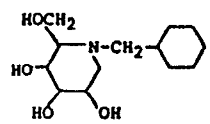

F.P.: 138—140°C (aus Aceton)

N-(3'-Cyclohexenylmethyl)-1-desoxynojirimycin

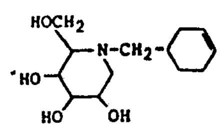

F.P.: 142—144°C (aus Aceton)

N-(2'-Norbornen-5'-yl-methyl)-1-desoxynojirimycin

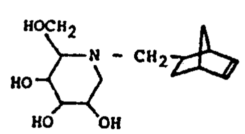

F.P.: 160—162°C (aus Ethanol)

N-p-Chlorbenzyl-1-desoxynojirimycin

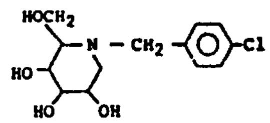

F.P.: 153—155°C (aus Aceton)

N-m-Methylbenzyl-1-desoxynojirimycin

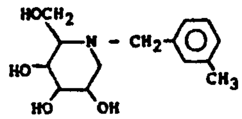

F.P.: 134—136°C (aus Methanol)

N-(p-Biphenylmethyl)-1-desoxynojirimycin

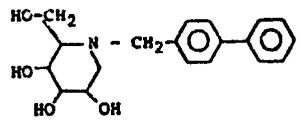

F.P.: 240—245°C (aus Wasser/Ethanol)

N-(n-3'-Phenylpropyl)-1-desoxynojirimycin

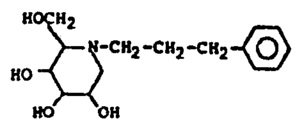

F.P.: 125—127°C (aus Ethanol)

Beispiel 4

N-Allyl-1-desoxynojirimycin

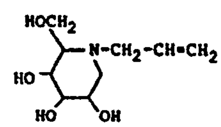

5 g 1-Desoxynojirimycin in 30 ml Dimethylformamid und 30 ml $H_2O$ wurden mit 5 g $Ag_2O$ und 5 g Allylbromid drei Stunden bei Raumtemperatur gerührt. Anschließend wurden die Silbersalze abfiltriert und das Filtrat wurde am Rotationsverdampfer zur Trockne gebracht. Der Rückstand wurde ans Ethanol umkristallisiert. Ausbeute: 4,5 g N-Allyl-1-desoxynojirimycin vom F.P. 131—132°C.

Auf analogem Wege wurden die folgenden Verbindungen hergestellt. Dabei erfolgte die Isolierung und Reinigung der Endprodukte gegebenenfalls auch durch eine Chromatographie über stark sauren Ionenaustauscher ($H^{\oplus}$-Form).

N-Propargyl-1-desoxyjirimycin

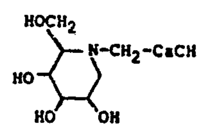

F.P.: 160°C (aus Aceton)

N-(3',4'-Dichlorbenzyl)-1-desoxynojirimycin

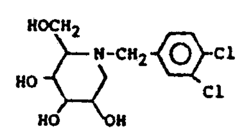

F.P.: 130—132°C

N-(p-Nitrobenzyl)-1-desoxynojirimycin

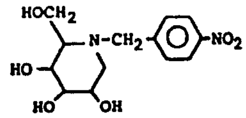

F.P.: 144—146°C

N-(m-Nitrobenzyl)-1-desoxynojirimycin

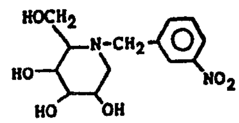

F.P.: 168—170°C

Beispiel 5

1-Cyano-1-desoxynojirimycin

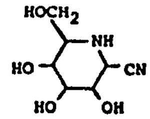

Zu 200 ml $H_2O$ und 21,2 g $Ba(OH)_2 \times 8\ H_2O$ gibt man 17,5 g Nojirimycinbisulfitaddukt. Man rührt eine Stunde bei Raumtemperatur und saugt den Feststoff ab. Das Filtrat versetzt man mit 12 ml flüssiger Blausäure und läßt 1/2 Stunde rühren. Die Lösung wird erneut filtriert und am Rotationsverdampfer bis auf 20 ml eingeengt. Man versetzt zunächst mit 20 ml MeOH, wobei das gewünschte Produkt auszukristallisieren beginnt und vervollständigt die Kristallisation durch Zugabe von 100 ml Ethanol. Der Niederschlag wurde abgesaugt.
Ausbeute: 12,0 g 1-Cyano-1-desoxynojirimycin; F.P.: 152—153°C. Nach Umkristallisation aus Methanol und wenig Wasser schmilzt die Substanz bei 155—156°C.

Beispiel 6

N-Methyl-1-cyano-1-desoxynojirimycin

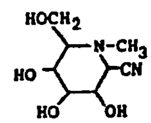

Die Verbindung wurde in Analogie zu Beispiel 3 durch reduktive Methylierung von 1-Cyano-1-desoxynojirimycin mit 35%-iger wäßriger Formaldehydlösung und $NaCNBH_3$ in Methanol erhalten.
*Massenspektrum:* Die wichtigsten Peaks im oberen Massenbereich liegen bei m/e = 171 (M—$CH_2OH$), m/e = 157 und m/e = 144.

Beispiel 7

1-Desoxynojirimycin-1-carbonsäure

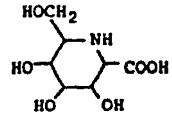

10 g 1-Cyano-1-desoxynojirimycin wurden mit 5 g NaOH in 100 ml $H_2O$ eine Stunde unter Rückfluß erhitzt. Anschließend wurde mit konz. HCl schwach sauer gestellt (pH=4). Dann wurde am Rotationsverdampfer zur Trockne eingeengt. Der Rückstand wurde mit Methanol in der Hitze extrahiert, das NaCl wurde abgetrennt und die methanolische Lösung erneut zur Trockne gebracht. Der Rückstand wurde zuerst aus wenig Wasser und dann aus Wasser/Methanol umkristallisiert.
Ausbeute: 10,5 g 1-Desoxynojirimycin-1-carbonsäure vom F.P.: 268—270°C.

Beispiel 8

1-Desoxynojirimycin-1-carbonsäureethylester

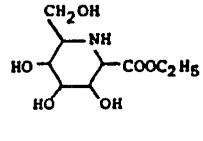

7 g 1-Desoxynojirimycin-1-carbonsäure wurden mit 100 ml ethanolischer Salzsäure 2 Stunden unter Rückfluß erhitzt. Anschließend wurde am Rotationsverdampfer zur Trockne eingeengt. Der Rückstand wurde mit Ethanol und zur Freisetzung der Base mit ethanolischem Ammoniak versetzt. Es wurde filtriert und die ethanolische Lösung wurde eingeengt.
Ausbeute an nichtkristallinem 1-Desoxynojirimycin-1-carbonsäureethylester: 8 g.

*NMR-Spektrum bei 100 MHz in $CD_3OD$:*

Triplett bei $\delta = 1{,}3$ ppm (3H, $—COO—CH_2—CH_3$);

Multiplett bei $\delta = 2{,}4—2{,}6$ ppm (1H, 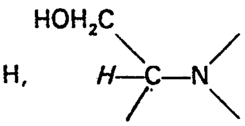

Multiplett bei $\delta = 3{,}2—3{,}5$ ppm (4H)

Multiplett bei $\delta = 3{,}6—3{,}9$ ppm (2H, $—CH_2OH$)

Quartett bei $\delta = 4{,}25$ ppm (2H, $—COO—CH_2—CH_3$)

Beispiel 9

N-Methyl-1-desoxynojirimycin-1-carbonsäureethylester

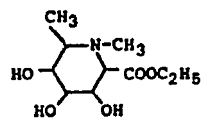

Aus 1-Desoxynojirimycin-1-carbonsäureethylester in Analogie zu Beispiel 6.
*Massenspektrum:* Die wichtigsten Peaks im oberen Massenbereich findet man bei: m/e = 218 ($M—CH_2OH$), m/e = 200, m/e = 176, m/e = 158 und m/e = 126.

Beispiel 10

1-Desoxynojirimycin-1-carbonsäureamid

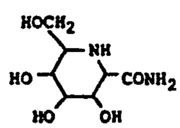

6 g 1-Desoxynojirimycin-1-carbonsäureethylester wurden in 90 ml 25%-igem wäßrigem Ammoniak eine Stunde unter Rückfluß erhitzt. Die abgekühlte Lösung wurde mit Ethanol versetzt und ein ausgefallener Niederschlag (1,2 g; Ammoniumsalz der 1-Desoxynojirimycin-1-carbonsäure) abgetrennt. Das Filtrat wurde eingeengt, in Wasser aufgenommen und auf eine mit stark basischem Austauscher in der $OH^{\ominus}$-Form (Amberlite IRC 400) gefüllte Säule aufgetragen. Es wurde mit Wasser eluiert. Die Fraktionen, die das Carbonsäureamid enthielten, wurden zusammengefaßt und eingeengt. Der Rückstand wurde aus Ethanol umkristallisiert. Ausbeute: 3 g 1-Desoxynojirimycin-1-carbonsäureamid von Schmelzpunkt 175—176°C.

Beispiel 11

1-Desoxynojirimycin-1-carbonsäurebenzylamid

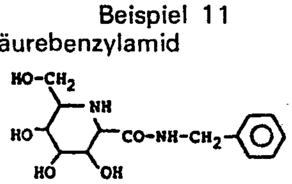

500 mg 1-Desoxynojirimycin-1-carbonsäureethylester wurden in 1 ml Benzylamin 5 Minuten zum Sieden erhitzt. Nach dem Abkühlen wurde mehrfach mit Ether verrührt und abdekantiert. Der Rückstand wurde aus Methanol umkristallisiert.
Ausbeute: 400 mg 1-Desoxynojirimycin-1-carbonsäurebenzylamid vom F.P. 221—222°C.

Beispiel 12

N-Methyl-1-desoxynojirimycin-1-carbonsäurebenzylamid

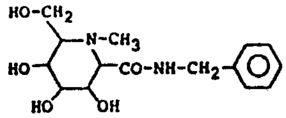

Aus 1-Desoxynojirimycin-1-carbonsäurebenzylamid in Analogie zu Beispiel 6.
F.P.: 229—230°C (aus Methanol).

Beispiel 13

1-Aminomethyl-1-desoxynojirimycin

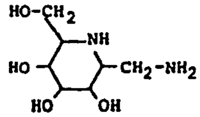

5 g 1-Cyano-1-desoxynojirimycin wurden in 100 ml Wasser mit 10 g Raney-Nickel als Katalysator eine Stunde bei 3,5 Athmosphären $H_2$-Druck in einer Schüttelbirne hydriert. Dann wurde vom Katalysator abgesaugt, die Lösung wurde am Rotationsverdampfer zur Trockne gebracht. Der Rückstand wurde in wenig siedendem Methanol aufgenommen, die Lösung wurde filtriert und erneut zur Trockne gebracht. Der Rückstand wurde aus ca. 15 ml Methanol umkristallisiert.
Ausbeute: 3,4 g 1-Aminomethyl-1-desoxynojirimycin vom Schmelzpunkt 148—150°C. Nach erneuter Kristallisation aus Methanol steigt der Schmelzpunkt auf 154—155°C.

Beispiel 14

1-Acetamidomethyl-1-desoxynojirimycin

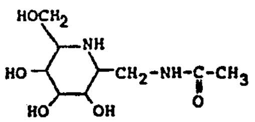

3,8 g 1-Aminomethyl-1-desoxynojirimycin in 40 ml $MeOH/H_2O$ 1:1 wurden bei 0°C mit 3 ml Acetanhydrid versetzt. Es wurde 15 Minuten bei 0°C und 30 Minuten bei Raumtemperatur gerührt. Dann wurde am Rotationsverdampfer zur Trockne eingeengt. Der Rückstand wurde in ca. 60 ml Wasser aufgenommen, und es wurde mit basischem Austauscher ($OH^{\ominus}$-Form) neutralisiert. Nach Entfernen des Austauschers wurde erneut zur Trockne eingeengt und der Rückstand zweimal aus Ethanol umkristallisiert.
Ausbeute an 1-Acetamidomethyl-1-desoxynojirimycin: 3 g vom F.P.: 169—171°C.
*MS-Spektrum:* Die wichtigsten Peaks im oberen Massenbereich findet man bei m/e = 216, m/e = 203, m/e = 162 (base peak) und m/e = 144.

Beispiel 15

N-Methyl-1-acetamidomethyl-1-desoxynojirimycin

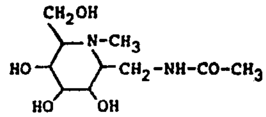

Die Verbindung wurde aus 1-Acetamidomethyl-1-desoxynojirimycin in Analogie zu Beispiel 6 hergestellt.
*MS-Spektrum:* Die wichtigsten Peaks im oberen Massenbereich findet man bei m/e = 176 und m/e = 158. Weniger intensive Peaks bei m/e = 230, m/e = 218 und 217.

Beispiel 16

1-Benzoylaminomethyl-1-desoxynojirimycin

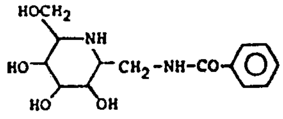

Die Verbindung wurde aus 1-Aminomethyl-1-desoxynojirimycin und Benzoylchlorid nach der Vorschrift von Beispiel 14 hergestellt.
F.P.: 216° (aus Methanol).
*Massenspektrum:* Die wichtigsten Peaks im oberen Massenbereich liegen bei m/e = 278, m/e = 265, m/e = 175, m/e = 162 und m/e = 105.

Beispiel 17

N-Methyl-1-benzoylaminomethyl-1-desoxynojirimycin

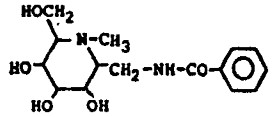

Die Verbindung wurde aus 1-Benzoylaminomethyl-1-desoxynojirimycin in Analogie zu Beispiel 6 hergestellt.

F.P.: 135—136°C (aus Butanol).

| Analyse: | | C | H | N |
|---|---|---|---|---|
| | *Ber.* | 58,1 | 7,1 | 9,0 |
| | *Gef.* | 57,2 | 7,3 | 9,0 |

Beispiel 18

1-Tosylamidomethyl-1-desoxynojirimycin

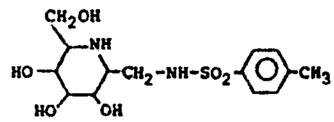

960 mg 1-Aminomethyl-1-desoxynojirimycin wurden mit 1 g Tosylchlorid in 10 ml $MeOH/H_2O$ 1:1 drei Stunden unter Rückfluß erhitzt. Anschließend wurde das Lösungsmittel am Rotationsverdampfer abgezogen, und der Rückstand wurde mit Aceton verrührt. Die Festsubstanz wurde abgesaugt, in Wasser gelöst und mit basischem Ionenaustauscher neutralisiert. Nach Entfernen des Ionenaustauschers wurde die Lösung am Rotationsverdampfer zur Trockne gebracht, und der Rückstand wurde aus Wasser umkristallisiert.

Ausbeute an 1-Tosylamidomethyl-1-desoxynojirimycin: 600 mg vom Schmelzpunkt: 173—175°C.

*NMR-Spektrum:*

Singlett bei $\delta = 2{,}36$ ppm (3H, $CH_3$ 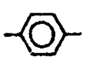 )

Multiplett bei $\delta = 2{,}4$—$2{,}7$ ppm (1H, $HOH_2C$

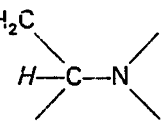

AA'BB'-System zwischen $\delta = 7{,}2$ und $7{,}8$ ppm (4H, 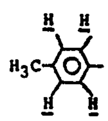 )

Die übrigen acht C—H-Protonen sind nicht einzeln zuzuordnen und absorbieren zwischen $\delta = 2{,}9$ und $\delta = 3{,}9$ ppm.

Beispiel 19

N-Methyl-1-tosylamidomethyl-1-desoxynojirimycin

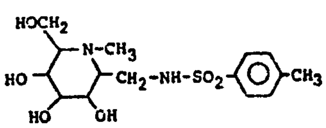

Die Verbindung wurde aus 1-Tosylamidomethyl-1-desoxynojirimycin nach der Vorschrift des Beispiels 6 hergestellt.

F.P.: 218—219°C (aus $H_2O$).

| Analyse: | | C | H | N |
|---|---|---|---|---|
| | Ber. | 50,0 | 6,7 | 7,8 |
| | Gef. | 49,7 | 6,6 | 8,1 |

Beispiel 20

1-(N'-Phenylureidomethyl)-1-desoxynojirimycin

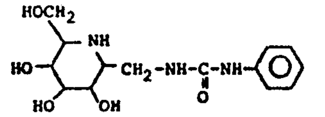

960 mg 1-Aminomethyl-1-desoxynojirimycin in 10 ml Methanol/Wasser 1:1 wurden bei –20°C mit 0,8 ml Phenylisocyanat 1/4 Stunde gerührt. Dann ließ man die Temperatur allmählich auf Raumtemperatur steigen. Nach Entfernen des Lösungsmittels wurde der Rückstand auf eine mit Cellulose gefüllte Säule aufgetragen. Es wurde mit Butanol, das 10% Wasser enthielt, eluiert. Die Fraktionen, die das Desoxynojirimycinderivat enthielten, wurden zusammengefaßt und eingeengt. Der Rückstand wurde aus Ethanol umkristallisiert.
Ausbeute: 400 mg 1-(N'-Phenylureidomethyl)-1-desoxynojirimycin vom Schmelzpunkt 161—162°C.
*Massenspektrum:* Die wichtigsten Peaks im oberen Massenbereich finden sich bei m/e = 218, m/e = 200 und m/e = 187, ein weiterer kleiner Peak bei m/e = 293.

Beispiel 21

N-(1-Desoxynojirimycin-yl)-essigsäurebenzylamid

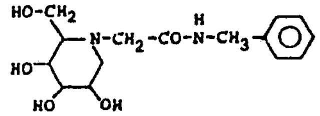

500 mg N-(1-Desoxynojirimycin-yl)-essigsäure-6-lacton wurden mit 1 ml Benzylamin in 20 ml DMF 6 Stunden unter Rückfluß erhitzt. Das Lösungsmittel wurde im Hochvakuum entfernt und der Rückstand aus Ethanol/Aceton 1:2 umkristallisiert.
Ausbeute: 400 mg N-(1-Desoxynojirimycin-yl)-essigsäurebenzylamid vom Schmelzpunkt 129°C.
*Massenspektrum:* Die wichtigsten Peaks im oberen Massenbereich finden sich bei m/e = 292, m/e = 279, m/e = 203 und m/e = 106.

Auf analogem Wege wurde hergestellt:

N-(1-Desoxynojirimycin-yl)-essigsäure-n-butylamid

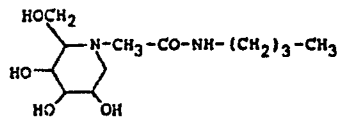

*Massenspektrum:* Die wichtigsten Peaks im oberen Massenbereich finden sich bei m/e = 245, m/e = 203, m/e = 176, m/e = 159 und m/e = 145.

Beispiel 22

1-Hydroxymethyl-1-desoxynojirimycin

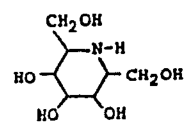

Zu 1,9 g $LiAlH_4$ in 50 ml absolutem THF wurde eine Suspension von 2,3 g 1-Desoxynojirimycin-1-carbonsäureethylester in 50 ml absolutem THF gegeben. Es wurde eine Stunde bei Raumtemperatur gerührt und dann 5 Stunden unter Rückfluß erhitzt. Anschließend wurden unter Rühren nacheinander 20 ml Essigester, 2 ml Wasser und 4 ml 15%-ige KOH zugetropft. Der ausgefallene Niederschlag wurde abgesaugt und mit einem Methanol/Wassergemisch extrahiert. Der Methanol/Wasserextrakt wurde am Rotationsverdampfer zur Trockne gebracht, und der Rückstand wurde mit Methanol extrahiert. Die methanolische Lösung wurde erneut eingeengt und der Rückstand mit Wasser auf eine mit stark saurem Austauscher in der $H^{\oplus}$-Form gefüllte Säule aufgetragen. Es wurde zuerst mit Wasser und dann mit 0,25%-igem Ammoniak eluiert. Die Fraktionen, die 1-Hydroxymethyl-1-desoxynojirimycin enthielten, wurden zusammengefaßt und eingeengt. Es wurden 500 mg 1-Hydroxymethyl-1-desoxynojirimycin erhalten.
*Massenspektrum:* Der wichtigste Peak (base peak) im oberen Massenbereich liegt bei m/e = 162. Kleinere Peaks findet man bei m/e = 144 und m/e = 102.

Beispiel 23

6-O-Benzoyl-1-desoxynojirimycin

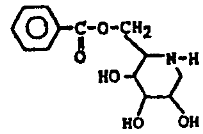

Zu 2,1 g 1-Desoxynojirimycin in 40 ml Aceton und 15 ml Wasser wurden bei Raumtemperatur 3,5 g gepulvertes $K_2CO_3$ und 2,0 g Benzoylchlorid gegeben. Es wurde 3 Stunden auf 40°C erwärmt und dann über Nacht bei Raumtemperatur gerührt. Anschließend wurde von Salzen abfiltriert und die Lösung im Vakuum eingeengt. Der Rückstand wurde an einer Kieselgelsäule chromatographiert. Es wurde zunächst mit Essigester/Methanol 10:4 und schließlich mit Essigester/Methanol/Wasser/Ammoniak 10:4:0,5:0,02 eluiert. Es wurden Fraktionen von jeweils 10 ml aufgefangen. Die Fraktionen 51—57 enthielten 350 mg 6-O-Benzoyl-1-desoxynojirimycin vom F.P. 160°C (aus Methanol).

Beispiel 24

N-(β-Methoxyethyl)-1-desoxynojirimycin

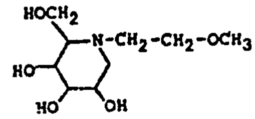

5,2 g β-Methoxyacetaldehyddimethylacetal in 15 ml $H_2O$ und 5 ml Methanol wurden mit 0,6 ml konz. HCl 48 Stunden bei Raumtemperatur und 6 Stunden bei 60°C hydrolysiert. Dann wurden bei Raumtemperatur 1,6 g 1-Desoxynojirimycin und 0,7 g $NaCNBH_3$ hinzugegeben. Man ließ über Nacht bei Raumtemperatur und dann noch 12 Stdn. bei 50°C reagieren. Das Reaktionsgemisch wurde im Vakuum zur Trockne gebracht, der Rückstand in Wasser auf eine mit stark saurem Ionenaustauscher in der $H^{\oplus}$-Form (Amberlite IR 120) gefüllte Säule aufgetragen. Es wurde zunächst mit Wasser, dann mit 2%-igem Ammoniak elueirt. Die Fraktionen, die N-(β-Methoxyethyl)-1-desoxynojirimycin enthielten, wurden zusammengefaßt und eingeengt. Der Rückstand wurde zur Trennung von wenig Ausgangsprodukt (1-Desoxynojirimycin) an einer Cellulosesäule chromatographiert. Als Fließmittel wurde Butanol/Wasser 9:1 verwendet.
Ausbeute an N-(β-Methoxyethyl)-1-desoxynojirimycin: 1,2 g.

Rf-Wert 0,57 (dünnschichtchromatographisch auf gebrauchsfertigen Silicagel 60-Platten der Fa. Merck, Laufmittel: Äthylacetat/Methanol/$H_2O$/25% Ammoniak 100:60:40:2).

Zum Vergleich Rf-Wert für 1-Desoxynojirimycin = 0,3.

Auf analogem Wege wurden erhalten:
N-(β-Methylmercaptoethyl)-1-desoxynojirimycin

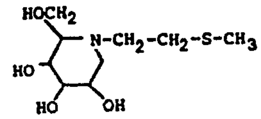

*Massenspektrum:* Die wichtigsten Peaks im oberen Massenbereich liegen bei m/e = 220, m/e = 206 und m/e = 176 (base peak).

N-(β-Ethylmercaptoethyl)-1-desoxynojirimycin

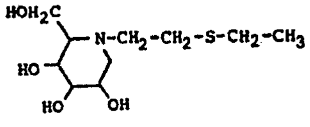

*Massenspektrum:* Die wichtigsten Peaks im oberen Massenbereich liegen bei m/e = 220 und m/e = 176 (base peak).

N-[β-(β'-Methoxy)-ethoxyethyl]-1-desoxynojirimycin

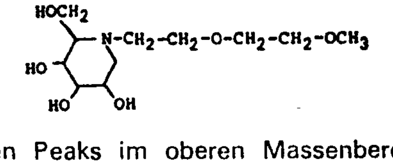

*Massenspektrum:* Die wichtigsten Peaks im oberen Massenbereich liegen bei m/e = 234 und

m/e = 176. Weitere Peaks findet man bei m/e = 218, m/e = 204, m/e = 158, m/e = 146 und m/e = 132.

Beispiel 25

N-n-Nonyl-1-acetamidomethyl-1-desoxynojirimycin

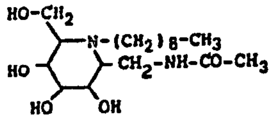

Die Verbindung wurde aus 1-Acetamidomethyl-1-desoxynojirimycin durch reduktive Alkylierung mit Nonylaldehyd und $NaCNBH_3$ in Methanol in Analogie zu Beispiel 3 erhalten.

*Massenspektrum:* Die wichtigsten Peaks des oberen Massenbereichs liegen bei m/e = 329, m/e = 288 (base peak), m/e = 270 und m/e = 258.

Beispiel 26

1-n-Nonylaminomethyl-1-desoxynojirimycin

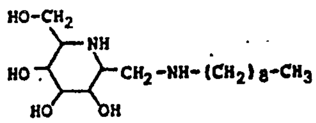

Zu 1,9 g 1-Aminomethyl-1-desoxynojirimycin in 40 ml Methanol wurden bei 0°C 1,2 ml Essigsäure, 1,56 g Nonylaldehyd und 0,7 g $NaCNBH_3$ gegeben. Es wurde eine Stunde bei 0°C und dann über Nacht bei Raumtemperatur gerührt. Dann wurde das Reaktionsgemisch am Rotationsverdampfer zur Trockne gebracht. Der Rückstand wurde in Wasser aufgenommen und auf eine mit stark saurem Ionenaustauscher in der $H^{\oplus}$-Form (Amberlite IR 120) gefüllte Säule aufgetragen. Die Säule wurde mit Ethanol/Wasser 1:1, dann mit 0,3%-igem wäßrigem Ammoniak und schließlich mit einem 1:1-Gemisch von Ethanol und 0.6%-igem wäßrigem Ammoniak eluiert. Die Fraktionen, die nach dünnschichtchromatographischer Überprüfung 1-n-Nonylaminomethyl-1-desoxynojirimycin enthielten, wurden zusammengefaßt und eigeengt.
Ausbeute: 1 g.

Rf-Wert: 0,52; 1-Desoxynojirimycin: 0,3. Platten und Laufmittel wie in Beispiel 25.

Beispiel 27

N-Methyl-nojirimycin-hydrochlorid

1. Herstellung der Ausgangsprodukte

In eine Lösung von 294,0 g (0,5 Mol) 3-O-Benzyl-6-O-triphenylmethyl-1.2-isopropyliden-5-amino-5-desoxy-$\alpha$-D-glucofuranose in 800 ml absolutem THF und 83,6 ml Triethylamin werden unter Eiskühlung 57 ml Chlorameisensäureethylester, gelöst in 360 ml absolutem THF, getropft. Es wird 2 Stdn. bei 20°C nachgerührt, vom ausgefallenen Salz abgesaugt und eingeengt. Es wird in Essigester aufgenommen, zweimal mit Wasser ausgeschüttelt, getrocknet und eingeengt. Man erhält 318,6 g (98,3% d.Th.) rohes 3-O-Benzyl-6-O-triphenylmethyl-1.2-O-isopropyliden-5-ethoxycarbonylamino-5-desoxy-$\alpha$-D-glucofuranose als leicht gelbes Öl.

174,7 g dieses Öles werden in 340 ml absolutem Ether gelöst und bei 10°C bis 15°C in eine Suspension von 39 g Lithium-aluminium-hydrid in 690 ml absolutem Ether getropft. Es wird anschließend 5 Stdn. zum Rückfluß erhitzt. Unter Eiskühlung wird danach durch Eintropfen von 520 ml Essigester, 40 ml Wasser und schließlich 78,5 ml 15%-iger Kalilauge zersetzt. Es wird abgesaugt, mit Ether gewaschen und i.V. eingedampft. Man erhält 144,2 g (91,3% d.Th.) 3-O-Benzyl-6-O-triphenylmethyl-1.2-isopropyliden-5-methylamino-5-desoxy-$\alpha$-D-glucofuranose als farbloses Öl.

Dieses Rohprodukt wird in 165 ml absolutem Tetrahydrofuran gelöst und bei −70°C in eine mit Trockeneis/Aceton gekühlte Mischung von 24,6 g Natrium in 820 ml flüssigem Ammoniak getropft. Es wird noch 2,5 g Natrium portionsweise zugegeben und 2 Stdn. nachgerührt. Dann wird bei −70°C portionsweise mit 91 g Ammonchlorid versetzt und über Nacht ohne Kältebad abrauchen gelassen. Die erhaltene Suspension wird mit 500 ml Methanol verrührt. Es wird abgesaugt und das Filtrat eingeengt. Der Eindampfrückstand in Wasser/Chloroform aufgenommen und getrennt. Die wäßrige Phase wird eingeengt. Das so erhaltene Rohprodukt wird über eine Austauscher-Säule Dowex 50 WX 4 gereinigt. Man erhält nach Umkristallisation aus Essigester 14,8 g (24,3% d.Th.) 5-Methylamino-5-desoxy-1.2.-O-isopropyliden-$\alpha$-D-glucofuranose vom Schmelzpunkt 124°C—126°C.

$C_{10}H_{19}NO_5$ (239,2) Ber.: C = 51,5 H = 8,15 N = 6,0

2. N-Methyl-nojirimycin (Hydrochlorid)

Eine Lösung von 470 mg 5-Methylamino-5-desoxy-1.2-O-isopropyliden-α-D-glucofuranose in 2 ml 6n HCl wird 16 Stdn. im Kühlschrank stehengelassen anschließend wird im Vakuum bei 20°C eingeengt, zweimal in Wasser gelöst und wieder eingeengt. Das auf diese Weise erhaltene nichtkristalline N-Methyl-nojirimycin-hydrochlorid zeigte im Saccharasehemmtest eine dreifach stärkere Wirkung als 1-Desoxy-nojirimycin.

Beispiel 28

N-Cyclohexyl-1-desoxy-nojirimycin

Methode A

2 g (12,25 mMol) 1-Desoxy-nojirimycin werden in 40 ml absolutem Methanol und 1,8 ml (30 mMol) Eisessig gelöst und nacheinander mit 5,2 ml (50 mMol) Cyclohexanon und 3,4 g (54 mMol) Natriumcyanoborhydrid versetzt. Diese Mischung wird 96 Stdn. zum Rückfluß erhitzt (DC-Kontrolle), abgekühlt und im Vakuum eingeengt. Der sirupose Eindampfrückstand wird in Methanol/Wasser 1:1 aufgenommen und über eine Austauschersäule mit Dowex 50 WX 4 H$^{\oplus}$-Form gereinigt. Man erhält 1,9 g reines Produkt.

Rf: 0,58; Essigester/Methanol/Wasser/25%iges Ammoniakwasser 120:70:10:1, DC-Platten Kieselgel 60 F 254 Merck Rf 1-Desoxy-nojirimycin: 0,13

Methode B

1 g 6-Cyclohexylamino-2.3-O-isopropyliden-6-desoxy-α-L-sorbofuranose vom Schmelzpunkt 95°C (hergestellt aus 1-O-Acetyl-2.3-O-isopropyliden-6-O-p-toluolsulfonyl-α-L-sorbofuranose durch Kochen mit Cyclohexylamin in Butanol) wird in einer Mischung von 6 ml Methanol/6n Salzsäure 1:1 bei 0°C 40 Stdn. stehengelassen, mit 10 ml Wasser verdünnt, mit 3,0 ml Triethylamin versetzt und nach Zugabe von Platindioxid 2 Stdn. bei 3,5 Bar hydriert. Es wird vom Katalysator filtriert, im Vakuum eingeengt und durch Austauscherchromatographie an Dowex 50 WX 4 gereinigt Man erhält 610 mg 1-Cyclohexyl-1-desoxy-nojirimycin, das identisch ist mit dem nach Methode A hergestellten Produkt.

Analog Methode A wurden hergestellt:

N-Isopropyl-1-desoxy-nojirimycin

Rf: 0,45

N-(1-Methyl-decyl)-1-desoxy-nojirimycin, Diastereomerengemisch Rf: 0,79 und 0,86

(Platten und Laufmittel wie in Beispiel 30 angegeben)

Beispiel 29

1.6-Desoxynojirimycin

(a) 5-Azido-3-O-benzyl-5.6-didesoxy-1.2.-O-isopropyliden-α-D-glucofuranose

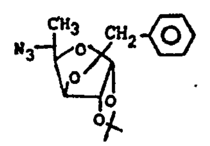

186 g (0,5 Mol) 3-O-Benzyl-6-desoxy-1.2-O-isopropyliden-5-O-methylsulfonyl-β-L-idofuranose (hergestellt nach: T.D. Inch, Carbohyd. Res. (1967), 45—52), 500 ml Dimethylsulfoxid und 65 g (1 Mol) $NaN_3$ wurden 5 Stdn. auf 120—125°C unter einem leichten Stickstoffstrom erhitzt. Nach dem Abkühlen wurde der Ansatz auf 500 ml Eiswasser gegeben, 3 x mit Petrolether extrahiert, die organische Phase mit Wasser gewaschen und über $Na_2SO_4$ getrocknet. Nach dem Einrotieren erhielt man 156 g (99%) der rohen 5-Azido-3-O-benzyl-5.6-didesoxy-1.2-O-isopropyliden-α-D-glucofuranose als Öl, die bei Bedarf durch Chromatographie an Kieselgel der Fa. Merck (70—230 mesh) mit Hexan/Ether 3:1 als Laufmittel rein dargestellt werden kann.

Charakteristische Banden im $^1$H—NMR (100 MHz, Lösungsmittel $C_6D_6$) sind: $\delta$ = 7.15 ppm (m, 5H), 5.72 ppm (d, J = 4Hz, 1H), 1.32 ppm (s, 3H), 1.17 ppm (d, J = 6Hz, 3H), 1.06 ppm (s, 3H).

(b) 5-Amino-3-O-benzyl-5.6-didesoxy-1.2-O-isopropyliden-α-D-glucofuranose

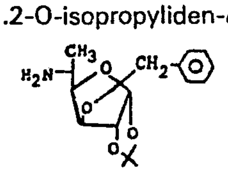

Man legte 6 g (0,156 Mol) $LiAlH_4$ in 250 ml wasserfreiem Tetrahydrofuran vor und tropfte bei Raumtemperatur 100 g (0,313 Mol) ungereinigte 5-Azido-3-O-benzyl-5.6-didesoxy 1.2-O-isopropyliden-α-D-glucofuranose in 200 ml wasserfreiem Tetrahydrofuran hinzu. Der Ansatz wurde

über Nacht bei Raumtemperatur gerührt und 1 Std. unter Rückfluß gekocht. Nach dem Abkühlen gab man unter Eiskühlung tropfenweise 6 ml Wasser und anschließend 18 ml 15%-ige KOH-Lösung hinzu, rührte über Nacht, saugte den Niederschlag ab und rotierte das Filtrat ein. Der Rückstand wurde in 500 ml Ether aufgenommen und zweimal mit 100 ml 2normaler Salzsäure extrahiert. Die wäßrige Phase stellte man mit 45%-iger NaOH-Lösung alkalisch und extrahierte dreimal mit je 200 ml Ether. Nach dem Trocknen der organischen Phase über $Na_2SO_4$ wurde das Lösungsmittel abrotiert. Man erhielt 62,5 g (68%) 5-Amino-3-O-benzyl-5.6-didesoxy-1.2-O-isopropyliden-$\alpha$-D-glucofuranose als Öl.

Das $^1H$—NMR (100 MHz, $CDCl_3$ als Lösungsmittel) zeigt folgende charakteristische Banden: $\delta$ = 7.3 ppm (m, 5H), 5.8 ppm (d, J = 4Hz, 1H), 5.70 ppm (d, J = 12Hz, 1H), 5.58 ppm (d, J = 4Hz, 1H), 5.42 ppm (d, J = 12Hz, 1H), 3.98 ppm (d, J = 4Hz), 1.45 ppm (s, 3H), 1.30 ppm (s, 3H), 1.15 ppm (d, J = 6Hz, 3H).

(c) 5-Amino-5.6-didesoxy-1.2-isopropyliden-$\alpha$-D-glucofuranose

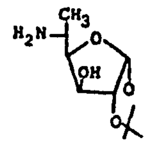

50 g (0,17 Mol) 5-Amino-3-O-benzyl-5.6-didesoxy-1.2-O-isopropyliden-$\alpha$-D-glucofuranose in 1 L Methanol wurden in Gegenwart von 10 g Palladium auf Kohle (5%-ig) bei 60°C unter einem Druck von 70 atm 5 Stdn. hydriert. Nach dem Abfiltrieren des Katalysators wurde das Lösungsmittel abrotiert. Ausbeute: 25,7 g (76%). Durch Umkristallisieren aus Essigester erhält man Kristalle.

Das $^1H$—NMR (100 MHz, Substanz gelöst in $CDCl_3$ und ausgeschüttelt mit $D_2O$): $\delta$ = 5.97 ppm (d, J = 4Hz, 1H), 4.50 ppm (d, J = 4Hz, 1H), 4.34 ppm (d, J = 4Hz, 1H), 1.49 ppm (s, 3H), 1.32 ppm (s, 3H), 1.28 ppm (d, J = 6Hz, 3H).

(d) 5-Amino-5.6-didesoxy-D-glucose-1-sulfonsäure

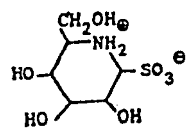

10 g (0,049 Mol) 5-Amino-5.6-didesoxy-1.2-O-isopropyliden-$\alpha$-D-glucofurose wurden in 50 ml Wasser suspendiert und 15 Stdn.

(e) 1.6-Didesoxynojirimycin

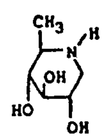

10 g (0,041 Mol) 5-Amino-5.6-didesoxy-D-glucose-1-sulfonsäure, 120 ml Wasser, 13,3 g $Ba(OH)_2 \times 8\,H_2O$ (0,042 Mol) und 10 g Raney-Nickel wurden bei Raumtemperatur und Normaldruck bis zum Ende der Wasserstoffaufnahme (etwa 7 Stdn.) hydriert. Der Ansatz wurde filtriert und das Filtrat i.Vak. eingedampft. Es blieb ein Öl zurück, das nach kurzer Zeit durchkristallisierte und sich aus Methanol umkristallisieren ließ.
Ausbeute: 5,3 g (83%), Fp. 163—164°C.

Als charakteristische Banden im $^1H$—NMR (100 MHz, Lösungsmittel $D_2O$) seien angeführt: $\delta$ = 3.24 ppm, 1.12 ppm (d, J = 6Hz).

Schwefeldioxid eingeleitet. Es entstand eine klare Lösung, worauf auf 60°C erwärmt wurde. Nach etwa 4 Stdn. begann die Abscheidung kristalliner 5-Amino-5.6-didesoxy-D-glucose-1-sulfonsäure. Zur Aufarbeitung versetzte man den Ansatz mit 100 ml Methanol, ließ über Nacht stehen und saugte den Niederschlag von 5-Amino-5.6-didesoxy-D-glucose-1-sulfonsäure ab.
Ausbeute: 8,5 g (71%), Fp. 180°C Zers.

Das $^1H$—NMR (100 MHz, Lösungsmittel DMSO-$d_6$ zeigt als charakteristische Bande bei $\delta$ = 1.13 ppm ein Dublett mit J = 6Hz.

## Beispiel 30

N-(1-Desoxyglucityl)-1-desoxynojirimycin

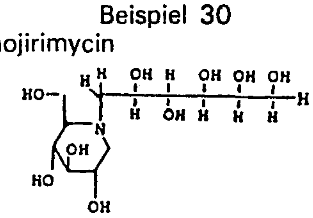

0,8 g (0,01 Mol) Desoxynojirimycin, 7,2 g (0,04 Mol) Glucose, 40 ml Methanol, 10 ml Wasser, 1,5 ml Eisessig und 1,3 g $NaBN_3CN$ wurden über Nacht bei Raumtemperatur gerührt und anschließend 6 Stdn. unter Rückfluß gekocht. Der Ansatz wurde i.Vak. zur Trockene eingedampft, mit 10 ml 2n HCl versetzt, bis zum Ende der Wasserstoffentwicklung auf 40°C erwärmt, auf eine Säule (30 cm lang, 2,5 cm Durchmesser) mit saurem Ionenaustauscher (Lewatit TWS 40) gegeben und mit 2 L Wasser nachgewaschen. Das Reaktionsprodukt wurde anschließend mit 0,3N $NH_3$-Lösung von der Säule eluiert, das Eluat i.Vak. eingedampft und der Rückstand an 100 g Kieselgel der Fa. Merck (70—230 mesh) mit Methanol/konz. Ammoniak-Lösung im Verhältnis 10:5 säulenchromatographisch gereinigt.
Ausbeute: 1 g.

Das Reaktionsprodukt läßt sich durch folgende Fragmente im Massenspektrum charakterisieren: m/e = 296 (20%), 278 (15%), 176 (100%), 158 (30%), 132 (30%).

Beispiel 31

1-Desoxy-6-O-methylnojirimycin

a) 3-O-Benzyl-1.2-O-isopropyliden-6-O-methyl-$\beta$-L-idofuranose

440 g 5,6-Anhydro-3-O-benzyl-1,2-O-isopropyliden-$\beta$-L-idofuranose 1,5 l Methanol und 92 g Natriummethylat wurden 1 Stunde unter Rückfluß gekocht. Nach dem Abkühlen wurde mit Eisessig neutralisiert, Methanol abdestilliert, der Rückstand auf 300 ml Wasser gegeben und mit Chloroform extrahiert. Nach dem Trocknen über Natriumsulfat und Abdestillieren des Lösungmittels blieben 388 g eines Öls.

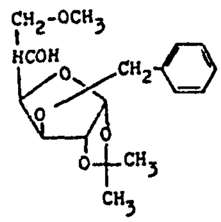

(1)

b) 3-O-Benzyl-1,2-O-isopropyliden-6-O-methyl-5-O-methylsulfonyl-$\beta$-L-idofuranose

Zu 384 g (1), 380 ml Pyridin und 760 ml Chloroform tropfte man bei 0°C 148 ml Methansulfonsäurechlorid und rührte über Nacht bei Raumtemperatur. Zur Aufarbeitung gab man 200 ml Eiswasser hinzu, rührte 20 Minuten und extrahierte dreimal mit je 200 ml Chloroform. Die organische Phase wurde zweimal mit verdünnter Salzsäure, anschließend mit Wasser und mit 10%iger Natriumhydrogencarbonatsösung gewaschen und über Natriumsulfat getrocknet. Nach dem Abdestillieren des Lösungsmittels im Vakuum wurde der Rückstand aus Essigester umkristallisiert. Man erhielt 347 g.

Aus der Mutterlauge konnte man nach Filtration über 200 g Kieselgel weitere 26 g isolieren.
Ausbeute: 79%
Schmelzpunkt: 133°C.

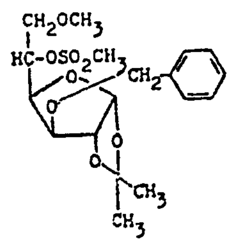

(2)

c) 5-Azido-3-O-benzyl-5-desoxy-1,2-O-isopropyliden-6-O-methyl-$\alpha$-D-glucofuranose

201 g (2) 500 ml Hexamethylphosphorsäuretriamid und 65 g Natriumazid wurden unter einem leichten Stickstoffatom über Nach auf 100—110°C erhitzt. Nach dem Abkühlen wurde die Mischung auf Eiswasser gegossen, 4 mal mit Äther extrahiert, die vereinigten Ätherextrakte mit verdünnter Salzsäure, Wasser und $NaHCO_3$-Lösung gewaschen, über $Na_2SO_4$ getrocknet im Vakuum eingedampft. Es blieben 159 g (91%) zurück.

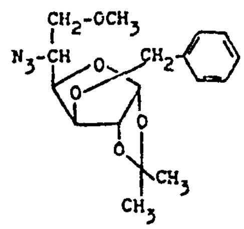

(3)

d) 5-Amino-3-O-benzyl-5-desoxy-1,2-O-isopropyliden-6-O-methyl-$\alpha$-D-glucofuranose

Zu 7,3 g $LiAlH_4$ in 500 ml wasserfreien Tetrahydrofuran (THF) tropfte man 134,5 g (3) in 200 ml wasserfreiem THF bei Raumtemperatur hinzu, rührte 4 Stunden und ließ den Ansatz über Nacht bei

Raumtemperatur stehen. Dann tropfte man 7,3 ml $H_2O$ langsam hinzu, versetzte anschließend mit 22 ml 15%iger KOH-Lösung und rührte 8 Stunden bei Raumtemperatur. Der Niederschlag wurde abgesaugt, mit THF gewaschen und das Filtrat im Vakuum eingedampft. Das erhaltene Öl wurde mit 300 ml Äther überschichtet und unter Eiskühlung bei 0—10°C mit 150 ml 5 N Salzsäure versetzt, die organische Phase abgetrennt, einmal mit verdünnter Salzsäure und die vereinigten wässrigen Phasen einmal mit Äther gewaschen. Anschließend versetzte man die wäßrige Phase mit 100 ml 40%iger NaOH-Lösung und extrahierte dreimal mit je 150 ml Äther. Die vereinigten Äther-Extrakte wurden über $Na_2SO_4$ getrocknet und im Vakuum eingedampft. Es blieben 91 g als Öl zurück.

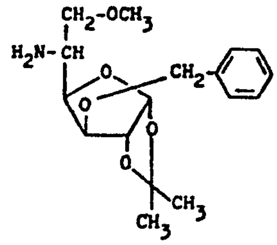

(4)

e) 5-Amino-5-desoxy-1,2-O-isopropyliden-6-O-methyl-α-D-glucofuranose

Zu 1,51 flüssigem Ammoniak gab man bei −70°C eine Lösung von 85 g (4) in 500 ml wasserfreiem THF und fügt anschließend 30,5 g Natrium in kleinen Stücken hinzu. Nach 4 Stunden wurde der Ansatz portionsweise vorsichtig mit insgesamt 106 g $NH_4Cl$ versetzt und anschließend über Nacht bei Raumtemperatur stehen gelassen, wobei der Ammoniak abdampfte. Der Rückstand wurde mit Methanol versetzt, der Niederschlag abgesaugt und das Filtrat im Vakuum eingedampft. Den Rückstand nahme man in Äther und verdünnter Salzsäure bei 0—10°C auf, extrahierte die Ätherphase dreimal mit insgesamt 300 ml verdünnter Salzsäure, versetzte die vereinigten Salzsäurephasen mit 200 ml konzentrierter Natronlauge und extrahierte dreimal mit insgesamt 600 ml Chloroform. Nach dem Trocknen des Chloroform-Extraktes über $Na_2SO_4$ wurde das Lösungsmittel im Vakuum abgedampft und der Rückstand aus Essigester umkristallisiert. Man erhielt 47 g (77%), Schmelzpunkt 95—96°C.

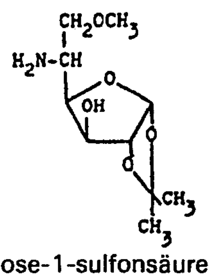

(5)

f) 5-Amino-5-desoxy-6-O-methyl-D-glucose-1-sulfonsäure

10 g (5) wurden in 50 ml Wasser gelöst. Dann wurde 2 Stunden bei Raumtemperatur, anschließend über Nacht bei 60°C $SO_2$ in die Lösung eingeleitet. Der Kristallbrei wurde mit 100 ml Methanol versetzt, 1 Tag bei Raumtemperatur stehen gelassen, der Niederschlag abgesaugt und über $CaCl_2$ im Vakuum getrocknet. Man erhielt 11,8 g (99%). Schmelzpunkt 154°C (unter Zersetzen)

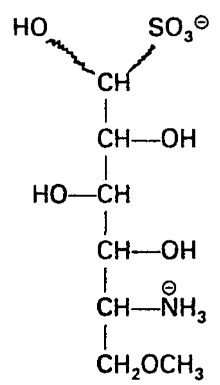

(6)

g) 1-Desoxy-6-O-methylnojirimycin

11 g (6) versetzte man in 90 ml Wasser mit 13.3 g $Ba(OH)_2 \cdot 8\ H_2O$. Das Produkt wurde ohne Isolierung in Gegenwart von 5 g Raney-Nickel unter Normaldruck bei Raumtemperatur 10 Stunden hydriert. Das Ungelöste wurde abfiltriert und die klare Lösung wurde im Vakuum eingedampft. Der Rückstand wurde in 30 ml 2 N Salzsäure aufgenommen und auf eine Säule mit saurem Ionenaustauscher gegeben. Es wurde mit 2 l Wasser nachgewaschen und mit 0,3 N Ammoniak-Lösung eluiert. Nach dem Eindampfen des Eluats im Vakuum blieb kristallines 1-Desoxy-6-O-methylnojirimycin zurück, das nach

Umkristallisieren aus Ethanol bei 145—146°C schmilzt. Ausbeute: 5,5 g (78%).

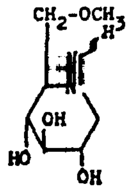

**Patentansprüche**

1. Verbindungen der Formel

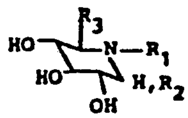

in der

$R_1$ H oder einen gegebenenfalls substituierten, geradkettigen, verzweigten oder cyclischen gesättigten oder ungesättigten aliphatischen Kohlenwasserstoffrest,

$R_2$ $-H$, $-OR'$, $-SR'$, $-N\begin{matrix}R'\\R''\end{matrix}$, $NR'R''-CH_2-$, $-COOR'$, $HO-CH_2-$, $R'CO-NR''CH_2-$,

$R'SO_2-NR''CH_2-$, $R'-NH-\underset{O}{\underset{\|}{C}}-NH-CH_2-$, $R'-NH-\underset{S}{\underset{\|}{C}}-NH-CH_2-$, $R'-O-\underset{O}{\underset{\|}{C}}-NH-CH_2-$,

$-SO_3H$, $-CN$, oder $-CONR'R''$,

$R_3$ H oder einen gegebenenfalls substituierten, geradkettigen, verzweigten oder cyclischen gesättigten oder ungesättigten aliphatischen Kohlenwasserstoffrest bedeuten,

wobei

R', R'' Wasserstoff, einen gegebenenfalls substituierten, geradkettigen verzweigten oder cyclischen gesättigten oder ungesättigten aliphatischen Kohlenwasserstoffrest oder einen gegebenenfalls substituierten aromatischen oder heterocyclischen Rest steht und wobei $R_1$ nicht Wasserstoff bedeutet, wenn

a) $R_2$ Wasserstoff oder OH und $R_3$ $CH_2OH$,

b) $R_2$ Wasserstoff, OH, $SO_3H$, CN oder $CH_2NH_2$ und $R_3$ Wasserstoff und

c) $R_2$ OH und $R_3$ $CH_2NH_2$ bedeuten,

und wobei $R_1$ nicht $C_1$—$C_4$-Alkyl bedeutet, wenn $R_2$ = H und $R_3$ = $CH_2OH$ ist, und deren pharmazeutisch annehmbaren Salze und bio-Vorläufer.

2. Verbindungen nach Anspruch 1 der Formel

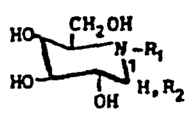

in der

$R_1$ H oder einen gegebenenfalls substituierten, geradkettigen, verzweigten oder cyclischen gesättigten oder ungesättigten aliphatischen Kohlenwasserstoffrest und

$R_2$ $-H$, $-OR'$, $-SR'$, $-N\begin{matrix}R'\\R''\end{matrix}$, $NR'R''-CH_2-$, $-COOR'$, $HO-CH_2-$, $R'CO-NR''CH_2-$,

$R'SO_2-NR''CH_2-$, $R'-NH-\underset{O}{\underset{\|}{C}}-NH-CH_2-$, $R'-NH-\underset{S}{\underset{\|}{C}}-NH-CH_2-$, $R'-O-\underset{O}{\underset{\|}{C}}-NH-CH_2-$,

$-SO_3H$, $-CN$, oder $-CONR'R''$ bedeutet,

wobei

R', R'' Wasserstoff, einen gegebenenfalls substituierten, geradkettigen, verzweigten oder cyclischen

gesättigten oder ungesättigten aliphatischen Kohlenwasserstoffrest oder einen gegebenenfalls substituierten aromatischen oder heterocyclischen Rest steht, und wobei $R_1$ nicht Wasserstoff ist, wenn $R_2$ Wasserstoff oder Hydroxy ist, und wobei $R_1$ nicht $C_1$—$C_4$-Alkyl bedeutet, wenn $R_2$ = H und $R_3$ = $CH_2OH$ ist.

3. Verbindungen gemäß Anspruch 1, bei denen $R_2$ für

—H, —OH, —$SO_3H$, —CN, —$CH_2NH_2$, —$CH_2NH_2$— [$C_1$—$C_6$-Alkyl] —$CH_2NH$—C(=O)— [$C_1$—$C_6$-Alkyl],

R'—NH—CO—NH—$CH_2$—, R'—NH—CS—NH—$CH_2$ oder R'—O—CO—NH—$CH_2$ und

$R_3$ für —H, —$CH_2OH$, —$CH_3$, —$CH_2NH_2$, —$CH_2$—NH—[$C_1$—$C_6$-Alkyl],

—$CH_2NH$—CO—$C_1$—$C_8$-Alkyl stehen.

4. N-(n-Heptyl)-1-desoxynojirimycin.

5. N-β-Hydroxyethyl-1-desoxynojirimycin.

6. Verfahren zur Herstellung von Verbindungen gemäß Anspruch 1, dadurch gekennzeichnet, daß man Verbindungen der Formel II oder IIa in der $R_1$ und $R_3$ die oben angegebene Bedeutung

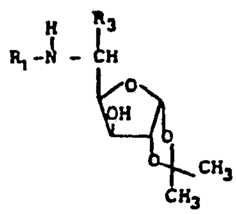

II

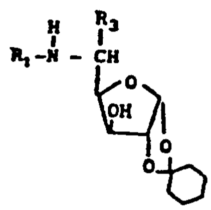

IIa

besitzen, zur Entfernung der Schutzgruppen der Säurehydrolyse unterwirft und die Verbindungen der Formel I mit $R_1$ = —OH als solche isoliert oder gegebenenfalls zu weiteren Verbindungen der Formel I umsetzt.

7. Verfahren zur Herstellung von Verbindungen gemäß Anspruch 1, dadurch gekennzeichnet, daß man Verbindungen der Formel V

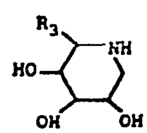

V

a) mit Carbonylverbindungen der Formel VI

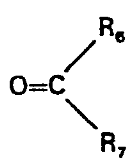

VI

in der

$R_6$, $R_7$ entweder H bedeuten oder die oben für $R_1$ angegebene Bedeutung besitzen oder Glieder eines alicyclischen oder heterocyclischen Ringes sind, in Gegenwart eines Wasserstoff-Donor-Reduktionsmittels umsetzt oder

b) mit reaktiven Alkylierungsmitteln der Formel IX

Z—$R_1$ IX

in der $R_1$ die oben für Alkyl angegebene Bedeutung besitzt und Z eine bei Alkylierungsmitteln gebräuchliche leicht austretende Gruppe darstellt, umsetzt und die Ansätze in üblicher Weise aufarbeitet, mit der Maßgabe, daß, wenn $R_3$ = $CH_2OH$ ist, $R_1$ in Z—$R_1$ nicht $C_1$—$C_4$-Alkyl ist und $R_6$ und $R_7$ keine Bedeutung annehmen, bei der $R_1$ im Reaktionsprodukt $C_1$—$C_4$-Alkyl ist.

**8. Arzneimittel, gekennzeichnet durch einen Gehalt an einer Verbindung gemäß Ansprüchen 1 bis 5 und gegebenenfalls pharmazeutisch geeigneten Zusatzstoffen.**

9. Verbindungen gemäß Ansprüchen 1 bis 5 zur Anwendung bei der Behandlung von Adipositas, Diabetes und/oder Hyperlipämie.

10. Tierfuttermittel, gekennzeichnet durch einen Gehalt an einer Verbindung gemäß den Ansprüchen 1 bis 5.

## Claims

1. Compounds of the formula

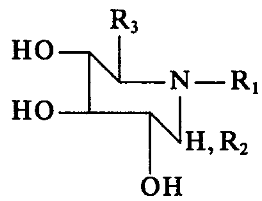

in which

$R_1$ denotes H or an optionally substituted, straight-chain, branched or cyclic saturated or unsaturated aliphatic hydrocarbon radical,
$R_2$ denotes

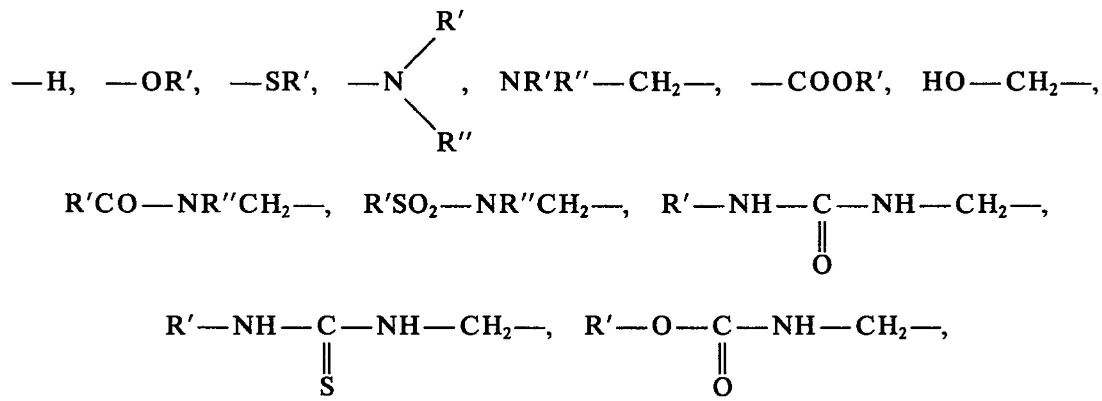

$-SO_2H$, $-CN$, or $-CONR'R''$,

$R_3$ denotes H or optionally substituted, straight-chain, branched or cyclic saturated or unsaturated aliphatic hydrocarbon radical,
wherein
R' and R'' represent hydrogen, an optionally substituted, straight-chain, branched or cyclic saturated or unsaturated aliphatic hydrocarbon radical or an optionally substituted aromatic or heterocyclic radical,

and wherein

$R_1$ does not denote hydrogen when
a) $R_2$ denotes hydrogen or OH and $R_3$ denotes $CH_2OH$,
b) $R_2$ denotes hydrogen, OH, $SO_3H$, CN or $CH_2NH_2$ and $R_3$ denotes hydrogen and
c) $R_2$ denotes OH an $R_3$ denotes $CH_2NH_2$,

and wherein

$R_1$ does not denote $C_1$—$C_4$-alkyl when $R_2$ is H and $R_3$ is $CH_2OH$, and pharmaceutically acceptable salts and bioprecursors thereof.

2. Compounds according to Claim 1, of the formula

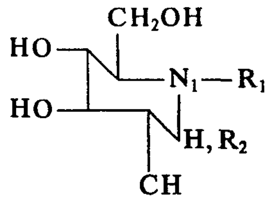

in which

$R_1$ denotes H or an optionally substituted, straight-chain, branched or cyclic saturated or unsaturated aliphatic hydrocarbon radical and
$R_2$ denotes

$-H$, $-OR'$, $-SR'$, 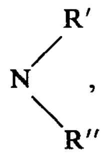 , $NR'R''-CH_2-$, $-COOR'$, $HO-CH_2-$,

$R'CO-NR''CH_2-$, $R'SO_2-NR''CH_2-$, $R'-NH-\underset{\underset{O}{\|}}{C}-NH-CH_2-$,

$R'-NH-\underset{\underset{S}{\|}}{C}-NH-CH_2-$, $R'-O-\underset{\underset{O}{\|}}{C}-NH-CH_2-$,

$-SO_2H$, $-CN$, or $-CONR'R''$

wherein

R' and R'' represent hydrogen, an optionally substituted, straight-chain, branched or cyclic saturated or unsaturated aliphatic hydrocarbon radical or an optionally substituted aromatic or heterocyclic radical,

and wherein

$R_1$ ist not hydrogen when $R_2$ is hydrogen or hydroxyl,

and wherein

$R_1$ does not denote $C_1-C_4$-alkyl when $R_2$ is H and $R_3$ is $CH_2OH$.

3. Compounds according to Claim 1, in which
$R_2$ represents

$-H$, $-OH$, $-SO_2H$, $CN$, $-CH_2NH_2$, $-CH_2NH-[C_1-C_6\text{-alkyl}]$

$-CH_2NH-\underset{\underset{O}{\|}}{C}-[C_1-C_6\text{-alkyl}]$.

$R'-NH-CO-NH-CH_2-$, $R'-NH-CS-NH-CH_2$ or

$R'-O-CO-NH-CH_2$

and

$R_3$ represents

$-H$, $-CH_2OH$, $-CH_2$, $-CH_2NH_2$, $CH_2-NH-[C_1-C_6\text{-alkyl}]$, or

$-CH_2NH-CO-C_1-C_6\text{-alkyl}$.

4. N-(n-Heptyl)-1-desoxynojirimycin.

5. N-β-Hydroxyethyl-1-desoxynojirimycin.

6. Process for the preparation of compounds according to Claim 1, characterised in that compounds of the formula II or II a in which $R_1$ and $R_3$ have the meaning given above

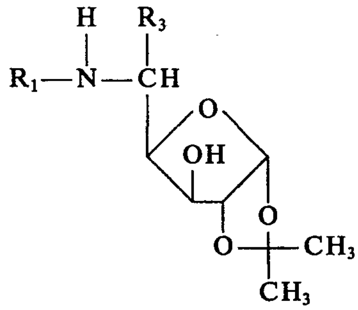

II

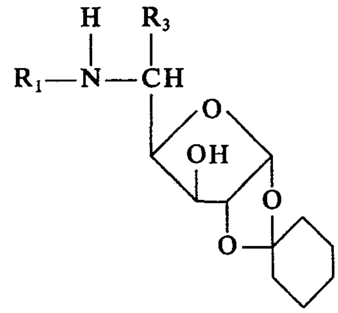

IIa

are subjected to acid hydrolysis to remove the protective groups and the compounds of the formula I which $R_1$ = — OH are isolated as such or are optionally reacted to give other compounds of the formula I.

7. Process for the preparation of compounds according to Claim 1, characterised in that compounds of the formula V

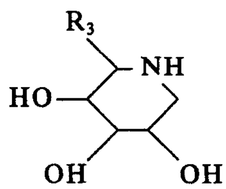

(V)

a) are reacted with carbonyl compounds of the formula VI

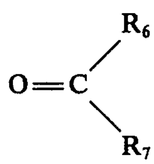

(VI)

in which
$R_6$ and $R_7$ either denote H or have the meaning given above for $R_1$ or are members of an alicyclic or heterocyclic ring, in the presence of a hydrogen donor reducing agent or

b) are reacted with reactive alkylating agents of the formula IX

$$Z—R_1 \qquad (IX)$$

in which
$R_1$ has the meaning given above for alkyl and
Z represents an easily eliminated leaving group which is customary in alkylating agents,

and the reaction products are worked up in the customary manner, with the proviso that when $R_3$ ist $CH_2OH$, $R_1$ in $Z—R_1$ is not $C_1 — C_4$-alkyl and $R_6$ and $R_7$ do not assume a meaning in which $R_1$ is $C_1 — C_4$-alkyl in the reaction product.

8. Medicaments, characterised in that they contain a compound according to Claims 1 to 5 and, if appropriate, pharmaceutically suitable additives.

9. Compounds according to Claims 1 to 5 for use in the treatment of adiposity, diabetes and/or hyperlipaemia.

10. Animal feedstuffs, characterised in that they contain a compound according to Claims 1 to 5.

**Revendications**

1. Composés de formule

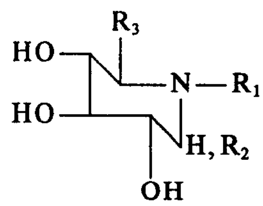

dans laquelle

$R_1$ représente H ou un reste d'hydrocarbure aliphatique saturé ou insaturé à chaîne droite, ramifié ou cyclique et éventuellement substitué,

$R_2$ représente

—H, —OR', —SR', 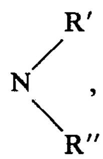, NR'R''—$CH_2$—, —COOR', HO—$CH_2$—,

R'CO—NR''$CH_2$—, R'$SO_2$—NR''$CH_2$—, 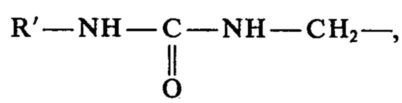,

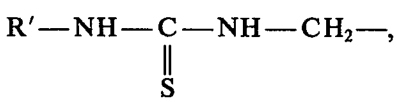, 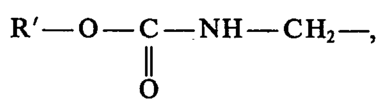,

—$SO_2$H, —CN, ou—CONR'R'',

$R_3$ représente H ou un reste d'hydrocarbure aliphatique saturé ou insaturé à chaîne droite, ramifié ou cyclique éventuellement substitué,

de sorte que

R', R'' représentent l'hydrogène, un reste d'hydrocarbure aliphatique saturé ou non saturé à chaîne droite, ramifié ou cyclique éventuellement substitué ou un reste aromatique ou hétérocyclique éventuellement substitué et $R_1$ ne représente pas l'hydrogène, lorsque

a) $R_2$ est l'hydrogène ou un groupe OH et $R_3$ est un groupe $CH_2OH$
b) $R_2$ est l'hydrogène, un groupe OH, $SO_3H$, CN ou $CH_2NH_2$ et $R_3$ est l'hydrogène et
c) $R_2$ représente OH et $R_3$ est un groupe $CH_2NH_2$,

et de sorte que $R_1$ ne représente pas un groupe alkyle en $C_1$ à $C_4$ lorsque $R_2$ représente H et $R_3$ représente un groupe $CH_2OH$,

et leurs sels et bioprécurseurs pharmaceutiquement acceptables.

2. Composés suivant la revendication 1, de formule

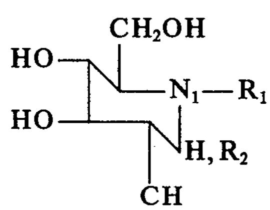

dans laquelle

$R_1$ représente H ou un reste d'hydrocarbure aliphatique saturé ou insaturé à chaîne droite, ramifié ou cyclique éventuellement substitué et

$R_2$ représente

$$-H,\quad -OR',\quad -SR',\quad N\genfrac{}{}{0pt}{}{\diagup R'}{\diagdown R''},\quad NR'R''-CH_2-,\quad -COOR',\quad HO-CH_2-,$$

$$R'CO-NR''CH_2-,\quad R'SO_2-NR''CH_2-,\quad R'-NH-\underset{\underset{O}{\|}}{C}-NH-CH_2-,$$

$$R'-NH-\underset{\underset{S}{\|}}{C}-NH-CH_2-,\quad R'-O-\underset{\underset{O}{\|}}{C}-NH-CH_2-,$$

$$-SO_2H, -CN, \text{ ou } -CONR'R'',$$

de sorte que

R', R'' représentent l'hydrogène, un reste d'hydrocarbure aliphatique saturé ou insaturé à chaîne droite, ramifié ou cyclique éventuellement substitué ou un reste aromatique ou hétérocyclique éventuellement substitué et de sorte que $R_1$ ne représente pas l'hydrogène lorsque $R_2$ représente l'hydrogène ou un groupe hydroxy, et que $R_1$ ne représente pas un groupe alkyle en $C_1$ à $C_4$ lorsque $R_2$ est l'hydrogène et $R_3$ est le groupe $CH_2OH$.

3. Composés suivant la revendication 1, dans lesquels $R_2$ représente

$$-H,\quad -OH,\quad -SO_2H,\quad CN,\quad -CH_2NH_2,\quad -CH_2NH-[C_1-C_6\text{-Alkyle}]$$

$$-CH_2NH-\underset{\underset{O}{\|}}{C}-[C_1-C_6\text{-Alkyle}].$$

$$R'-NH-CO-NH-CH_2-,\quad R'-NH-CS-NH-CH_2 \text{ ou}$$

$$R'-O-CO-NH-CH_2$$

et

$R_3$ représente

$$-H,\quad -CH_2OH,\quad -CH_2,\quad -CH_2NH_2,\quad CH_2-NH-[C_1-C_6\text{-Alkyle}],$$

$$-CH_2NH-CO-C_1-C_6\text{-Alkyle}.$$

4. La N-(n-heptyl)-1-désoxynojirimycine.

5. La N-$\beta$-hydroxyéthyl-1-désoxynojirimycine.

6. Procédé de production de composés suivant la revendication 1, caractérisé en ce qu'on soumet à l'hydrolyse acide pour éliminer les groupes protecteurs des composés de formule II ou de formule II a dans laquelle $R_1$ et $R_3$ ont la définition indiquée ci-dessus

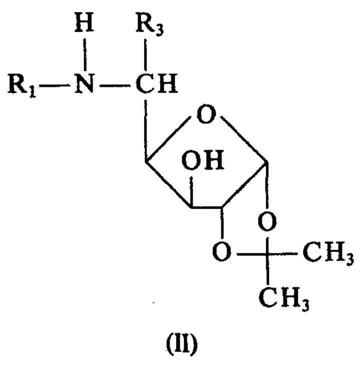

(II)

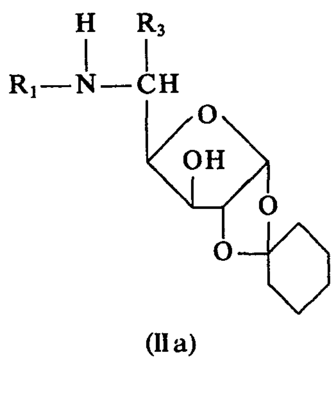

(II a)

et on isole tels quels les composés de formule I dans laquelle $R_1$ représente — OH ou bien on les fait réagir le

cas échéant pour former d'autres composés de formule I.

7. Procédé de production de composés suivant la revendication 1, caractérisé en ce qu'on fait réagir des composés de formule V

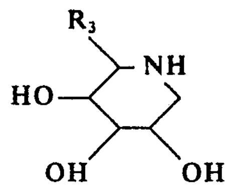

(V)

a) avec des composés carbonyliques de formule VI

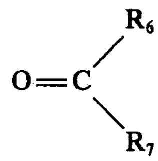

(VI)

dans laquelle

$R_6$, $R_7$ représentent H ou possèdent la définition indiquée ci-dessus pour $R_1$ ou sont des membres d'un noyau alicyclique ou hétérocyclique, en présence d'un agent réducteur cédant de l'hydrogène, ou bien

b) avec des agents alkylants réactifs de formule IX

$$Z—R_1 \qquad (IX)$$

dans laquelle $R_1$ a la définition indiquée ci-dessus pour alkyle et Z représente un groupe aisément partant d'emploi classique dans le cas d'agents alkylants et on traite les mélanges réactionnels de manière classique, sous réserve que lorsque $R_3$ représente $CH_2OH$, $R_1$ dans Z — $R_1$ ne soit pas un groupe alkyle en $C_1$ à $C_4$ et que $R_6$ et $R_7$ n'admettent pas de définition selon laquelle $R_1$ est un groupe alkyle en $C_1$ à $C_4$ dans le produit réactionnel.

8. Médicament, caractérisé par une teneur en un composé suivant les revendications 1 à 5 et le cas échéant en additifs utilisables en pharmacie.

9. Composés suivant les revendications 1 à 5 destinés à être utilisés dans le traitement de l'adiposité, du diabète et/ou de l'hyperlipidémie.

10. Aliment pour les animaux, caractérisé par une teneur en un composé suivant les revendications 1 à 5.